# EUROPEAN PATENT APPLICATION

(11) **EP 1 323 734 A1**
(43) Date of publication of application: **02.07.2003**
(21) Application number: 01956969.8
(22) Date of filing: 20.08.2001
(51) Int. Cl.: C07K 14/47, C12N 15/12, A61K 31/711, A61P 3/08, A61P 3/10, A61K 38/17, G01N 33/50, G01N 33/15

(54) **IRAP-BINDING PROTEIN**

(30) Priority: 21.08.2000 JP 2000254263; 07.09.2000 JP 2000276633
(71) Applicant: Takeda Chemical Industries, Ltd., Osaka-shi, Osaka 541-0045 (JP)
(72) Inventor: TOJO, Hideaki, Ushiku-shi, Ibaraki 300-1207 (JP); KATAYAMA, Nozomu, Tsukuba-shi, Ibaraki 305-0821 (JP); KAKIMOTO, Shigeya, Tsukuba-shi, Ibaraki 305-0074 (JP)
(74) Representative: Lewin, John Harvey
(86) International application number: JP0107117
(87) International publication number: WO02016428

(57) **Abstract**

It is an object of the present invention to provide an IRAP-binding protein. Specifically, the present invention provides the IRAP-binding protein, pharmaceuticals comprising the protein, a method for screening a compound inhibiting binding of the protein to IRAP, and a compound obtained by the screening method.

The protein according to the present invention is useful as prophylactic and/or therapeutic agents for diseases such as hypoglycemia. Moreover, the protein according to the present invention is useful as a reagent for screening a compound inhibiting a binding of the protein according to the present invention to IRAP (insulin responsive aminopeptidase) or GLUT4 (glucose transporter 4). The compound inhibiting the binding of the protein according to the present invention to IRAP or GLUT4 is useful as prophylactic and/or therapeutic agents for diseases such as hyperglycemia and diabetes.

## Description

### Technical Field

The present invention relates to a protein or a salt thereof biding to insulin responsive aminopeptidase (IRAP) or glucose transporter 4 (GLUT4), a method for screening a prophylactic and/or therapeutic agent for hyperglycemia or diabetes by using the protein or a salt thereof, a compound obtained by the screening method, and a use of the compound.

### Background Art

Blood glucose level is regulated by an action of insulin through intake of glucose in a skeletal muscle and an adipose tissue. Diabetes is caused by continuation of a high blood glucose level through decrease in this action. Intake of glucose into a cell requires involvement of a membrane protein called a glucose transporter (glucose transporting carrier). At present, 8 species, GLUT1-8, have been known in the glucose transporter (Bell et al., J. Biol. Chem. 268: 3352-3356. 1993; Olson and Pessi, Annu. Rev. Nutr., 16: 235-256. 1996; Ibberson et al. J. Biol. Chem. 275: 4607-4612. 2000; Dodge et al. J. Biol. Chem. 275: 16275-16280). That, which is involved strongly in a sugar transporting activity of insulin, among these glucose transporters as described above is GLUT4, which is expressed mainly in the skeletal muscle and the adipose tissue (Fukumoto et al. Proc. Natnl Acad. Sci. USA 85: 53: 5434-5438. 1988; Birnbaum et al. Cell 57: 305-315. 1989).

Usually, GLUT4 is present in intracellular vesicles called GLUT4 vesicles in a cell and at an increasing sugar level, it is translocated to a cell membrane by the action of insulin to enhance intake of sugar (Bell et al., Diabetes Care 13: 198-208. 1990; Czech et al, Trans Biochem. Sci., 17: 197-201. 1992).

To elucidate a molecular mechanism of translocation of GLUT4 vesicles, not only GLUT4 itself, but also other proteins constituting GLUT4 vesicles have been studied for identification. At present, molecules known as constituting GLUT4 vesicles are as follows: VMAPS (vesicle-associated membrane proteins: Cain et al., J. Biol. Chem. 267: 11681-11634. 1992), SCAMPs (secretory component- associated membrane proteins. Thiodis et al. J. Biol. Chem. 268: 11681-11696. 1993; Laurie et al. J. Biol. Chem. 268: 19110-19117. 1993), phophatidylinositol 4-kinase (Del Vacchio & Pilch, J. Biol. Chem. 266: 13278-13283. 1991), low molecular weight GTP-binding protein Rab4 (Cormont et al. J. Biol. Chem. 268: 19491-19497. 1993), and also IRAP (insulin-responsive aminopeptidase: Kandror & Pilch, Proc. Natnl Acad. Sci. USA 91: 8017-8021. 1994; Kandror et al. J. Biol. Chem. 269: 30777-30780. 1994; Keller et al. J. Biol. Chem. 270: 23612-23618. 1995).

IRAP is called also gp160 being a membrane protein of once transmembrane and present locally in GLUT4 vesicles of cell organelles. As to protein structure, an intracytoplasmic domain consists of 109 amino acids located in an amino terminal (N terminal) followed by a transmembrane domain consisting of 22 amino acids, and an extracellular domain consisting of 785 amino acids located in a carboxyl terminal (C terminal) (Kandror & Pilch, Proc. Natnl Acad. Sci. USA 91: 8017-8021. 1994; and Keller et al. J. Biol. Chem. 270: 23612-23618. 1995). The extracellular domain is a zinc-dependent protease (amino peptidase) of which activity has been found (Kandror et al. J. Biol. Chem. 269: 30777-30780. 1994). Since injecting a peptide, which corresponds to the N terminal domain (intracytoplasmic domain) among these domains, into a cell causes translocation on the GLUT4 vesicles to a cell surface, it has been predicted that a protein binding to IRAP is present to clamp the GLUT4 vesicles to the intracellular region (Walters et al. J. Biol. Chem. 272: 23323-23327. 1997.)

A sequence of a cDNA obtained by the present invention coincides with the sequence reported for a human long-chain acyl-CoA dehydrogenase (VLCAD) (Anderson et al. Hum. Mol. Benet. 5: 461-472. 1996). There is no report on the fact that the protein binds to IRAP or directly participates in blood sugar regulation.

### Disclosure of the Invention

The present invention provides a method for screening a compound having a hypoglycemic action with an application of an interaction of proteins between VLCAD and IRAP, the compound obtained by the screening method and the like.

As the result of our intensive studies, the inventors successfully cloned VLCAD as the IRAP-binding protein from cDNA library derived from a human skeletal muscle by using yeast two-hybrid method (Fields and Strenglanz. Trans. Genet. 10: 286-292. 1994; Brent & Finley. Annu. Rev. Genet. 31: 663-704. 1997). The inventors further studied resulting in the present invention.

The present invention concerns:
(1) A protein or a salt thereof having an amino acid sequence identical or substantially identical with the amino acid sequence represented by SEQ ID NO: 1, which is bound to an insulin responsive aminopeptidase or glucose transporter 4.
(2) A pharmaceutical comprising the protein or the salt thereof according to (1).
(3) The pharmaceutical comprising a DNA containing the DNA encoding the protein according to (1).
(4) The pharmaceutical according to (2) or (3), being a binder to the insulin responsive aminopeptidase or glucose transporter 4.
(5) The pharmaceutical according to (2) or (3), being a prophylactic and/or therapeutic agent for hypoglycemia.
(6) A diagnostic agent comprising the DNA containing the DNA encoding the protein according to (1).
(7) A method for prevention and treatment of hypoglycemia, which comprises administering an effective amount of the protein or the salt thereof according to (1) to a nonhuman mammal.
(8) Use of the protein or the salt thereof according to (1) for manufacturing the prophylactic and/or therapeutic agent for hypoglycemia.
(9) The pharmaceutical comprising an antisense DNA containing a base sequence or a segment thereof, which is complementary or substantially complementary to the base sequence of the DNA encoding the protein according to (1).
(10) The pharmaceutical comprising an antibody against the protein or the salt thereof according to (1).
(11) The pharmaceutical according to (9) or (10), being a prophylactic and/or therapeutic agent for hyperglycemia or diabetes.
(12) A method for prevention and/or treatment of hyperglycemia or diabetes, which comprises administering the effective amount of the antibody against the protein or the salt thereof according to (1) to the nonhuman mammal.
(13) Use of the antibody against the protein or the salt thereof according to (1) for manufacturing the prophylactic and/or therapeutic agent for hyperglycemia or diabetes.
(14) A diagnostic agent comprising the antibody against the protein or the salt thereof according to (1).
(15) A method for screening a compound having a hyperglycemic action or a hypoglycemic action, or the salt thereof, which comprises using the protein containing the amino acid sequence identical or substantially identical with the amino acid sequence represented by SEQ ID NO: 1,a partial peptide thereof, or the salt thereof.
(16) The method for screening the compound having the hyperglycemic action or the hypoglycemic action, or the salt thereof, which comprises using the DNA containing the DNA, which encodes the protein containing the amino acid sequence identical or substantially identical with the amino acid sequence represented by SEQ ID NO: 1, or the partial peptide thereof.
(17) A kit for screening the compound having the hyperglycemic action or the hypoglycemic action, or the salt thereof, wherein the kit comprises the protein containing the amino acid sequence identical or substantially identical with the amino acid sequencerepresented by SEQ ID NO: 1, the partial peptide thereof, or the salt thereof.
(18) The kit for screening the compound having the hyperglycemic action or the hypoglycemic action, or the salt thereof, wherein the kit comprises the DNA containing the DNA, which encodes the protein containing the amino acid sequence identical or substantially identical with the amino acid sequence represented by SEQ ID NO: 1, or the partial peptide.
(19) The compound having the hyperglycemic action, or the salt thereof obtainable by employing the screening method according to (15) or (16) or the screening kit according to (17) or (18).
(20) A pharmaceutical comprising the compound according to (19) or the salt thereof.
(21) The pharmaceutical according to (20), which is the prophylactic and/or therapeutic agent for hypoglycemia.
(22) The compound having the hypoglycemic action, or the salt thereof obtainable by employing the screening method according to (15) or (16) or the screening kit according to (17) or (18).
(23) A pharmaceutical comprising the compound according to (22), or the salt thereof
(24) The pharmaceutical according to (23), which is a prophylactic and therapeutic agent for hyperglycemia or diabetes.
(25) A method for screening the compound or the salt thereof enhancing or inhibiting binding the protein containing the amino acid sequence identical or substantially identical with the amino acid sequence represented by SEQ ID NO: 1, the partial peptide thereof, or the salt thereof to the insulin responsive aminopeptidase or glucose transporter 4, which comprises using the protein containing the amino acid sequence identical or substantially identical with the amino acid sequence represented by SEQ ID NO: 1,the partial peptide thereof, or the salt thereof.
(26) The screening method according to (25), which comprises using a cell having an ability to produce the protein containing the amino acid sequence identical or substantially identical with the amino acid sequence represented by SEQ ID NO: 1,or the partial peptide thereof.
(27) The screening method according to (25), which comprises adding a test compound to a complex between (1) the protein, containing the amino acid sequence identical or substantially identical with the amino acid sequence represented by SEQ ID NO: 1, the partial peptide thereof, or the salt thereof, and (2) the insulin responsive aminopeptidase or glucose transporter 4 labeled, of which one has been fixed on a solid phase, and detecting a free substance.
(28) The screening method according to (25), which comprises culturing a cell transformed with (1) DNA encoding the partial peptide corresponding to a cytoplasmic domain of the insulin responsive aminopeptidase, or the partial peptide corresponding to a intracellular domain of glucose transporter 4, with which a reporter gene binding domain has been fused, and (2) DNA encoding the protein or the partial peptide thereof containing the amino acid sequence identical or substantially identical with the amino acid sequence represented by SEQ ID NO: 1, with which a reporter gene transcription-active domain has been fused, in a presence and absence of the test compound and detecting expression of a reporter gene in both the cases.
(29) The screening method according to (28), wherein the partial peptide corresponding to the cytoplasmic domain of the insulin responsive aminopeptidase is the partial peptide containing the amino acid sequence from 55th to 82nd position of the amino acid sequence represented by SEQ ID NO: 5 and the partial peptide corresponding to the cytoplasmic domain of glucose transporter 4 is the partial peptide containing the amino acid sequence represented by SEQ ID NO: 7.
(30) A kit for screening the compound or the salt thereof, which contains the protein, the partial peptide thereof, or the salt thereof containing the amino acid sequence identical or substantially identical with the amino acid sequence represented by SEQ ID NO: 1, inhibiting the binding of the protein, the partial peptide thereof, or the salt thereof containing the amino acid sequence identical or substantially identical with the amino acid sequence represented by SEQ ID NO: 1, to the insulin responsive aminopeptidase or glucose transporter 4.
(31) A compound or the salt thereof, which is obtainable by using the screening method according to (25) to (29) or the screening kit according to (30), inhibiting the binding of the protein, the partial peptide thereof, or the salt thereof containing the amino acid sequence identical or substantially identical with the amino acid sequence represented by SEQ ID NO: 1, to the insulin responsive aminopeptidase or glucose transporter 4.
(32) A pharmaceutical comprising the compound or the salt thereof according to (31).
(33) The pharmaceutical according to (32), which is a prophylactic and/or therapeutic agent for hyperglycemia or diabetes.
(34) A method for prevention and treatment of hyperglycemia or diabetes, which comprises administering the effective amount of the compound or the salt thereof according to (31) to the nonhuman mammal.
(35) Use of the compound or the salt thereof according to (31), for manufacturing the prophylactic and/or therapeutic agent for hyperglycemia or diabetes.
(36) A compound or the salt thereof, which is obtainable by using the screening method according to (25) to (29) or the screening kit according to (30), enhancing the binding of the protein, the partial peptide thereof, or the salt thereof containing the amino acid sequence identical or substantially identical with the amino acid sequence represented by SEQ ID NO: 1, to the insulin responsive aminopeptidase or glucose transporter 4.
(37) A pharmaceutical comprising the compound or the salt thereof according to (36).
(38) The pharmaceutical according to (37), which is a prophylactic and/or therapeutic agent for hypoglycemia.
(39) A method for prevention and treatment of hypoglycemia, which comprises administering the effective amount of the compound or the salt thereof according to (36) to the nonhuman mammal.
(40) Use of the compound or the salt thereof according to (31), for manufacturing the prophylactic and/or therapeutic agent for hypoglycemia.
(41) A method for screening the compound enhancing or suppressing the expression of the protein, or the salt thereof, which comprises using the protein or the salt thereof containing the amino acid sequence identical or substantially identical with the amino acid sequence represented by SEQ ID NO: 1.
(42) The screening method according to (41), which comprises culturing the cell transformed with DNA, which is prepared by fusing the reporter gene with a transcription regulating region of DNA encoding the protein containing the amino acid sequence identical or substantially identical with the amino acid sequence represented by SEQ ID NO: 1 in the presence and absence of the test compound and detecting expression of the reporter gene in each case.
(43) A kit for screening the compound enhancing or suppressing the expression of the protein, or the salt thereof, wherein the kit comprises the protein or the salt thereof containing the amino acid sequence identical or substantially identical with the amino acid sequence represented by SEQ ID NO: 1.
(44) A compound or the salt thereof, which is obtainable by using the screening method according to (41) or (42), or the screening kit according to (43), suppressing expression of the protein containing the amino acid sequence identical or substantially identical with the amino acid sequence represented by SEQ ID NO: 1.
(45) A pharmaceutical comprising the compound or the salt thereof according to (44).
(46) The pharmaceutical according to (45), which is the prophylactic and/or therapeutic agent for hyperglycemia or diabetes.
(47) A compound or the salt thereof, which is obtainable by using the screening method according to (41) or (42), or the screening kit according to (43), enhancing expression of the protein containing the amino acid sequence identical or substantially identical with the amino acid sequence represented by SEQ ID NO: 1.
(48) The pharmaceutical comprising the compound or the salt thereof according to (47).
(49) The pharmaceutical according to (48), which is a prophylactic and/or therapeutic agent for hypoglycemia.
(50) A method for prevention and treatment of hypoglycemia, which comprises administering the effective amount of the compound or the salt thereof according to (47) to the nonhuman mammal.
(51) Use of the compound or the salt thereof according to (47) for manufacturing the prophylactic and/or therapeutic agent for hypoglycemia.

### Brief Description of the Drawings

Fig. 1 shows a base sequence and a predicted amino acid sequence of human VLCAD (MD25) gene (cDNA) (continuing to Fig. 2).
Fig. 2 shows the base sequence and the predicted amino acid sequence of human VLCAD (MD25) gene (cDNA) (continued from Fig. 1 and continuing to Fig. 3).
Fig. 3 shows the base sequence and the predicted amino acid sequence of human VLCAD (MD25) gene (cDNA) (continuing to Fig. 2).
Fig. 4 shows an interaction of IRAP with VLCAD on the basis of quantifying a β-galactosidase activity. In the figure, - of bait expresses GAL4-DNABD sequence only and IRAP expresses the sequence prepared by fusing this with IRAP (55-82). On the other hand, - of prey expresses a control without any prey vector and MD-25 expresses the sequence prepared by fusing GAL4-AD with base sequence from 118th base of VLCAD. In IRAP/ MD-25, the β-galactosidase activity is significantly observed. A value of the result is expressed with a β-galactosidase activity unit (mean ± s.d.)
Fig. 5 shows a distribution of MD25 mRNA in tissues. In the figure, brain, heart, skeletal muscle, colon, thymus, spleen, kidney, liver, small intestine, placenta, lung and leukocyte shows nou, sinzou, kokkaku-kin, kecchou, kyousen, hizou, jinzou, kanzou, shouchou, taiban, hai and hakkekkyuu in Japanese, respectively. A size (kb) of RNA is indicated on the left side.

### BEST MODE FOR CARRYING OUT THE INVENTION

The protein having an amino acid sequence identical or substantially identical with an amino acid sequence represented by SEQ ID NO: 1 of the present invention (hereafter referred to as "protein according to the present invention") may be any protein derived from any cells of human and other mammals (e.g., guinea pig, rat, mouse, fowl, rabbit, swine, sheep, bovine, monkey, etc.), for example, hepatic cell, spleen cells, nerve cells, glial cells, β-cells of pancreas, bone marrow cells, mesangial cells, mesangial cell, Langerhans' cells, epidermal cells, epithelial cells, beaker cell, endothelial cells, smooth muscle cell, fibroblasts, fibrocytes, muscular cells, adipose cells, immunocytes (e.g., macrophage, T cells, B cells, natural killer cells, mast cells, neutrophils, basophils, eosinophils or monocytes), megakaryocytes, synovial cells, chondrocytes, osteocytes, osteoblasts, osteoclasts, mammary gland cells, hepatocytes or interstitial cells, or precursor cells, stem cells or cancer cells of these cells, and the like; hemocytes; or any tissues containing such cells, for example, brain or various parts of the brain (e.g., olfactory bulb, amygdala, cerebral basal ganglia, hippocampus, thalamus, hypothalamus, cerebral cortex, medulla oblongata, cerebellum), spinal cord, pituitary, stomach, pancreas, kidney, liver, genital gland, thyroid gland, gallbladder, bone marrow, adrenal gland, skin, muscle, lung, digestive tract (e.g., large intestine, small intestine), blood vessel, heart, thymus, spleen, submandibular gland, peripheral blood, prostate, testicle, testis, ovary, placenta, uterus, bone, joint, skeletal muscle and the like. The protein may also be synthetic.

The amino acid sequence which has the identical or substantially identical to the amino acid sequence represented by SEQ ID NO: 1 includes an amino acid sequence having about 70% homology, preferably at least about 80% homology, more preferably at least about 90% homology, much more preferably at least about 99% homology, most preferably at least about 95% homology, to the amino acid sequence represented by SEQ ID NO: 1.

A preferred example of the protein containing the substantially identical amino acid sequence to the amino acid sequence represented by SEQ ID NO: 1 is a protein containing substantially identical amino acid sequence to the amino acid sequence represented by SEQ ID NO: 1 and having an activity substantially equivalent to that of the amino acid sequence represented by SEQ ID NO: 1.

As the substantially equivalent property, there is, for example, a binding activity to IRAP or GLUT4. The term substantially equivalent is used to mean that these activities are qualitatively equivalent in nature to one another. It is thus preferred that the binding activity to IRAP or GLUT4 is equivalent, but quantitative factors such as the degree of these properties, a molecular weight of protein, etc. may be different from each other.

An amino acid sequence substantially identical to the amino acid sequence represented by SEQ ID NO: 1 of the present invention is more specifically exemplified by the amino acid sequence represented by SEQ ID NO: 9, the amino acid sequence represented by SEQ ID NO: 10, the amino acid sequence represented by SEQ ID NO: 11, and the like.

As the protein of the present invention, for example, there may be used a protein, so-called mutein, containing (i) an amino acid sequence represented by SEQ ID NO: 1, SEQ ID NO: 9, SEQ ID NO: 10, or SEQ ID NO: 11, in which 1, 2 or more amino acids (preferably about 1 to about 25 amino acids, more preferably about 1 to about 10 amino acids, most preferably several (1 to 5) amino acids) are deleted; (ii) an amino acid sequence represented by SEQ ID NO: 1, SEQ ID NO: 9, SEQ ID NO: 10, or SEQ ID NO: 11, to which 1, 2 or more amino acids (preferably about 1 to about 25 amino acids, more preferably about 1 to about 10 amino acids, most preferably several (1 to 5)) are added; (iii) an amino acid sequence represented by SEQ ID NO: 1, SEQ ID NO: 9, SEQ ID NO: 10, or SEQ ID NO: 11, in which 1, 2 or more amino acids (preferably about 1 to about 25 amino acids, more preferably about 1 to about 10 amino acids, most preferably several (1 to 5)) are inserted; (iv) an amino acid sequence represented by SEQ ID NO: 1, SEQ ID NO: 9, SEQ ID NO: 10, or SEQ ID NO: 11, in which 1, 2 or more amino acids (preferably about 1 to about 25 amino acids, more preferably about 1 to about 10 amino acids, most preferably several (1 to 5)) are substituted by other amino acids; or (v) the protein, having a combination of the amino acid sequences described above.

The proteins in the present specification are designated according to the established practice of describing peptides, in which the left end is the N-terminal (amino terminal) and the right end is the C-terminal (carboxyl terminal). In the proteins of the present invention including the protein containing the amino acid sequence represented by SEQ ID No: 1, the C-terminal is normally a carboxyl group (-COOH) or a carboxylate (-COO⁻), but the C-terminal may be an amide (-CONH₂) or an ester (-COOR).

Herein, examples of the ester group shown by R include a C₁₋₆ alkyl group such as methyl, ethyl, n-propyl, isopropyl, n-butyl, etc.; a C₃₋₈ cycloalkyl group such as cyclopentyl, cyclohexyl, etc.; a C₆₋₁₂ aryl group such as phenyl, α-naphthyl, etc.; a C₇₋₁₄ aralkyl group such as a phenyl-C₁₋₂ alkyl group, e.g., benzyl, phenethyl, etc.; an α-naphthyl-C₁₋₂ alkyl group, e.g., α-naphthylmethyl, etc.; and the like. In addition, pivaloyloxymethyl or the like which is used widely as an ester for oral administration may also be used.

Where the protein of the present invention contains a carboxyl group (or carboxylate) at a position other than the C-terminal, it may be amidated or esterified and such an amide or ester is also included within the protein of the present invention. The ester group may be the same group as that described with respect to the above C-terminal.

Further, the protein of the present invention includes derivatives wherein the amino group (e. g., methionine residue) of the N-terminal of the above protein is protected with a protecting group (e.g., a C₁₋₆ acyl group such as a C₁₋₆ alkanoyl group such as formyl group, acetyl group, etc.); derivatives wherein the N-terminal region is cleaved in vivo and the glutamyl group produced is pyroglutaminated; and derivatives wherein a substituent (e.g., -OH, -SH, -COOH, amino group, imidazole group, indole group, guanidino group, etc.) on the side chains of an amino acid in the molecule of the protein is protected with an appropriate protecting group (e.g., a C₁₋₆ acyl group such as a C₁₋₆ alkanoyl group such as formyl group, acetyl group, etc.), or conjugated proteins such as glycoproteins having sugar chains.

Specific examples of the protein of the present invention that can be used include a protein, VLCAD described on a reference (Andersen et al. Hum. Mo. Benet. 5: 461-472. 1996), which is derived from a human skeletal muscle, containing the amino acid sequence represented by SEQ ID NO: 1, and the like.

The partial peptide of the protein of the present invention (hereafter sometimes merely referred to as the partial peptide of the present invention) may be any peptide, as long as it is a partial peptide of the protein of the present invention described above, preferably, any one of them if their properties satisfy those of the protein of the present invention described above. For example, the peptides or the like preferably used have amino acid sequences of at least 20 or more, preferably 50 or more, more preferably 70 or more, much more preferably 100 or more, and most preferably 200 or more amino acid sequences of the protein of the present invention. Particularly preferably, the peptide used has amino acid sequences constituting of 200 or more and less than 655 (more preferably 200 or more and less than 620) amino acid residues continuing from the C terminal of the protein of the present invention.

The partial peptides of the present invention may be those wherein 1, 2 or more amino acids (preferably approximately 1 to 10 amino acids, more preferably several (1 to 5) amino acids) may be deleted in the amino acid sequence described above; 1, 2 or more amino acids (preferably approximately 1 to 10 amino acids, more preferably several (1 to 5) amino acids) may be added to the amino acid sequence; or, 1, 2 or more amino acids (preferably approximately 1 to 10 amino acids, more preferably several, most preferably approximately 1 to 5) amino acids) may be inserted into the amino acid sequence; or, 1, 2 or more amino acids (preferably approximately 1 to 10 amino acids, more preferably several, most preferably approximately 1 to 5) amino acids) may be substituted by other amino acids in the amino acid sequence.

The partial peptides of the present invention are more specifically exemplified by the partial peptides containing the amino acid sequence represented by the sequence from 36th (Ala) to 655th (Phe) starting from the N terminal of the amino acid sequence represented by SEQ ID NO. 1.

In the partial peptides of the present invention, the C-terminal is normally a carboxyl group (-COOH) or a carboxylate (-COO⁻) but the C-terminal may be an amide (-CONH₂) or an ester (-COOR), as has been described with respect to the protein of the present invention.

As in the protein of the present invention described above, the partial peptide of the present invention also includes those wherein the amino group (e.g., methionine residue) of the amino acid residue of the N-terminal is protected by a protecting group, those wherein the N-terminal residue is cleaved in vivo and the produced Glutamine is converted into pyroglutamate, those wherein substituents on the side chains of amino acids in the molecule are protected by appropriate protecting groups and conjugated peptides to which sugar chains are bound, that is glycopeptides, and the like.

The partial peptide of the present invention can be used as an antigen for preparation of an antibody, and also used for screening a compound inhibiting the binding of the protein of the present invention to IRAP or GLUT4.

The salts of the protein or its partial peptide of the present invention include physiologically acceptable salts with acids (e. g., organic acids and organic acids) or bases (e. g., alkali metal salts,) and in particular, physiologically acceptable acid addition salts are preferred. Examples of such salts are salts with inorganic acids (e.g., hydrochloric acid, phosphoric acid, hydrobromic acid, sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The protein or the partial peptide of the present invention or salts thereof may be manufactured by publicly known methods for purification of proteins from the human or other warm-blooded animal cells or tissues described above. Alternatively, the protein of the present invention or salts thereof may also be manufactured by culturing a transformant containing a DNA encoding the protein or the peptide of the present invention, as will be later described. Furthermore, the protein of the present invention or salts thereof may also be manufactured by the method for peptide synthesis, which will also be described hereafter.

When the protein or the partial peptide according to the present invention or salts thereof are manufactured from human or other mammal tissues or cells, the human or other mammalian tissues or cells are homogenized and extracted with an acid or the like, and the extract yielded is isolated and purified by a combination of chromatography techniques such as reversed phase chromatography, ion exchange chromatography, and the like.

To synthesize the protein or its partial peptide thereof of the present invention, or salts or amides thereof, commercially available resins that are used for protein synthesis can be used. Examples of such resins include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmehtylphenyl acetamidomethyl resin, polyacrylamide resin, 4-(2',4'-dimethoxyphenyl-hydroxymethyl) phenoxy resin, 4-(2',4'-dimethoxyphenyl-Fmoc-aminoethyl) phenoxy resin, etc. Using these resins, amino acids wherein α-amino groups and functional groups on the side chains are appropriately protected are condensed on the resin in the order of the sequence of the objective protein or peptide, following various condensation methods publicly known in the art. At the end of the reaction, the protein or the peptide is excised from the resin and at the same time, the protecting groups are removed. Then, intramolecular disulfide bond-forming reaction is performed in a highly diluted solution to obtain the objective protein or amides thereof.

For condensation of the protected amino acids described above, a variety of activation reagents usable for protein synthesis may be employed, but carbodiimides are particularly preferably used. Examples of such carbodiimides include DCC, N,N'-diisopropylcarbodiimide, N-ethyl-N'-(3-dimethylaminopropyl)carbodiimide, etc. For activation by these reagents, the protected amino acids are added directly to the resin together with a racemization inhibitor (e.g., HOBt, HOOBt), or the protected amino acids are previously activated in the form of symmetric acid anhydrides, HOBt esters or HOOBt esters, followed by adding the thus activated protected amino acids to the resin.

Solvents suitable for use in the activation of protected amino acids or in the condensation with resins may be selected from solvents that are known to be usable for protein condensation reactions. Examples of such solvents are acid amides such as N,N-dimethylformamide, N,N-dimethylacetamide, N-methylpyrrolidone, etc.; halogenated hydrocarbons such as methylene chloride, chloroform, etc.; alcohols such as trifluoroethanol, etc.; sulfoxides such as dimethylsulfoxide, etc.; ethers such as pyridine, dioxane, tetrahydrofuran, etc.; nitriles such as acetonitrile, propionitrile, etc.; esters such as methyl acetate, ethyl acetate, etc.; or appropriate mixtures of these solvents, and the like. The reaction temperature is appropriately chosen from the range known to be applicable to protein bond forming reactions and is usually selected from the range of approximately -20°C to 50°C. The activated amino acid derivatives are used generally in an excess of 1.5 to 4 times. The condensation is examined using the ninhydrin reaction; when the condensation is insufficient, the condensation can be completed by repeating the condensation reaction without removal of the protecting groups. When the condensation is yet insufficient even after repeating the reaction, unreacted amino acids are acetylated with acetic anhydride or acetylimidazole to cancel any possible adverse effect on the subsequent reaction.

Examples of the protecting groups used to protect the starting amino groups include Z, Boc, tertiary-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc, etc.

A carboxyl group can be protected by, e.g., alkyl esterification (in the form of linear, branched or cyclic alkyl esters of methyl, ethyl, propyl, butyl, t-butyl, cyclopentyl, cyclohexyl, cycloheptyl, cyclooctyl, 2-adamantyl, etc.), aralkyl esterification (e.g., benzyl ester, 4-nitrobenzyl ester, 4-methoxybenzyl ester, 4-chlorobenzyl ester, and benzhydryl ester), phenacyl esterification, benzyloxycarbonyl hydrazidation, t-butoxycarbonyl hydrazidation, trityl hydrazidation, etc.

The hydroxyl group of serine can be protected by, for example, its esterification or etherification. Examples of groups appropriately used for the esterification include a lower (C₁₋₆) alkanoyl group such as acetyl group, etc., an aroyl group such as benzoyl group, etc., a group derived from carbonic acid such as benzyloxycarbonyl group, ethoxycarbonyl group, etc. Examples of a group appropriately used for the etherification include benzyl group, tetrahydropyranyl group, t-butyl group, etc.

Examples of groups for protecting the phenolic hydroxyl group of tyrosine include Bzl, Cl₂-Bzl, 2-nitrobenzyl, Br-Z, t-butyl, etc.

Examples of groups used to protect the imidazole moiety of histidine include Tos, 4-methoxy-2,3,6-trimethylbenzenesulfonyl, DNP, benzyloxymethyl, Bum, Boc, Trt, Fmoc, etc.

Examples of the activated carboxyl groups in the starting amino acids include the corresponding acid anhydrides, azides, activated esters [esters with alcohols (e.g., pentachlorophenol, 2,4,5-trichlorophenol, 2,4-dinitrophenol, cyanomethyl alcohol, p-nitrophenol, HONB, N-hydroxysuccimide, N-hydroxyphthalimide, HOBt)], etc. As the activated amino acids in which the amino groups are activated in the starting material, the corresponding phosphoric amides are employed.

To eliminate (split off) the protecting groups, there are used catalytic reduction under hydrogen gas flow in the presence of a catalyst such as Pd-black, Pd-carbon, etc.; an acid treatment with anhydrous hydrogen fluoride, methanesulfonic acid, trifluoromethanesulfonic acid or trifluoroacetic acid, or a mixture solution of these acids, etc.; a treatment with a base such as diisopropylethylamine, triethylamine, piperidine, piperazine, etc.; reduction with sodium in liquid ammonia, or the like. The elimination reaction of the protecting group by the acid treatment described above is carried out generally at a temperature of approximately -20°C to 40°C. In the acid treatment, it is efficient to add a cation scavenger such as anisole, phenol, thioanisole, m-cresol, p-cresol, dimethylsulfide, 1,4-butanedithiol, 1,2-ethanedithiol, etc. Furthermore, 2,4-dinitrophenyl group known as the protecting group for the imidazole of histidine is removed by a treatment with thiophenol. Formyl group used as the protecting group of the indole of tryptophan is eliminated by the aforesaid acid treatment in the presence of 1,2-ethanedithiol, 1,4-butanedithiol, etc. as well as by a treatment with an alkali such as a dilute sodium hydroxide solution, dilute ammonia, etc.

Protection of functional groups that should not be involved in the reaction of the starting materials, protecting groups, elimination of the protecting groups and activation of functional groups involved in the reaction may be appropriately chosen from publicly known groups and publicly known means.

In another method for obtaining the amidated protein or the peptide, for example, the α-carboxyl group of the carboxy terminal amino acid is first protected by amidation; the peptide (protein) chain is then extended from the amino group side to a desired length. Thereafter, a protein or a peptide in which only the protecting group of the N-terminal α-amino group has been eliminated from the peptide and a protein or a peptide in which only the protecting group of the C-terminal carboxyl group has been eliminated are manufactured. The two proteins or peptides are condensed in a mixture of the solvents described above. The details of the condensation reaction are the same as described above. After the protected protein or peptide obtained by the condensation is purified, all the protecting groups are eliminated by the method described above to give the desired crude protein or peptide. This crude protein or peptide is purified by various known purification means. Lyophilization of the major fraction gives the amide of the desired protein or peptide.

To prepare the objective esterified protein or peptide, for example, the α-carboxyl group of the carboxy terminal amino acid is condensed with a desired alcohol to prepare the amino acid ester, which is followed by procedure similar to the preparation of the amidated protein above to give the desired esterified protein.

The partial peptide of the present invention or salts thereof can be manufactured by publicly known methods for peptide synthesis, or by cleaving the protein of the present invention with an appropriate peptidase. For the methods for peptide synthesis, for example, either solid phase synthesis or liquid phase synthesis may be used. That is, the partial peptide or amino acids that can construct the partial peptide of the present invention are condensed with the remaining part (peptide or amino acid). Where the product contains protecting groups, these protecting groups are removed to give the desired peptide. Publicly known methods for condensation and elimination of the protecting groups are described in 1) - 5) below.
1) M. Bodanszky & M.A. Ondetti: Peptide Synthesis, Interscience Publishers, New York (1966)
2) Schroeder & Luebke: The Peptide, Academic Press, New York (1965)
3) Nobuo Izumiya, et al.: Peptide Gosei-no-Kiso to Jikken (Basics and experiments of peptide synthesis), published by Maruzen Co. (1975)
4) Haruaki Yajima & Shunpei Sakakibara: Seikagaku Jikken Koza (Biochemical Experiment) 1, Tanpakushitsu no Kagaku (Chemistry of Proteins) IV, 205 (1977)
5) Haruaki Yajima, ed.: Zoku lyakuhin no Kaihatsu (A sequel to Development of Pharmaceuticals), Vol. 14, Peptide Synthesis, published by Hirokawa Shoten

After completion of the reaction, the product may be purified and isolated by a combination of conventional purification methods such as solvent extraction, distillation, column chromatography, liquid chromatography, recrystallization and the like to give the partial peptide of the present invention. When the protein or peptide obtained by the above methods is in a free form, the peptide can be converted into an appropriate salt by a publicly known method or a similar method; when the protein is obtained in a salt form, it can be converted into a free form or any other salts by a publicly known method or a similar method.

The DNA encoding the protein of the present invention may be any DNA so long as it contains the base sequence encoding the protein of the present invention described above. The DNA encoding the protein of the present invention may be any of genomic DNA, genomic DNA library, cDNA derived from the cells and tissues described above, cDNA library derived from the cells and tissues described above and synthetic DNA.
The vector to be used for the library may be any of bacteriophage, plasmid, cosmid and phagemid. The DNA may be directly amplified by reverse transcriptase polymerase chain reaction (hereafter abbreviated as RT-RCR) using the total RNA or mRNA fraction prepared from the cells and tissues described above.

The DNA encoding the protein of the present invention may be any DNA so long as it has, for example, DNA having the base sequence represented by SEQ ID NO: 2 or it has a base sequence hybridizable to DNA having the base sequence represented by SEQ ID NO: 2 under high stringent conditions and encodes a protein having a substantially equivalent activity as that of the protein of the present invention. Examples of the DNA that is hybridizable to DNA having the base sequence represented by SEQ ID NO: 2 under the high stringent condition include a DNA having the base sequence having at least about 70% homology and preferably at least about 99% homology, to the base sequence represented by SEQ ID NO: 2.

The hybridization can be carried out by publicly known methods or by modifications of these methods, for example, according to the method described in Molecular Cloning, 2nd Ed.; J. Sambrook et al., Cold Spring Harbor Lab. Press, (1989). A commercially available library may also be used according to the instructions of the attached manufacturer's protocol. The hybridization can be carried out preferably under high stringent conditions.

The high stringent conditions used herein are, for example, those in a sodium concentration of about 19 to about 40 mM, preferably about 19 to about 20 mM and a temperature at about 50 to about 70°C, preferably about 60 to about 65°C. In particular, hybridization conditions in a sodium concentration at about 19 mM and a temperature at about 65°C are most preferred.

More specifically, for the DNA encoding the protein containing the amino acid sequence represented by SEQ ID NO: 1, there may be employed a DNA containing the base sequence represented by SEQ ID NO: 2.

The DNA encoding the partial peptide of the present invention may be any DNA so long as it contains the base sequence encoding the protein of the present invention described above. The DNA encoding the protein of the present invention may be any of genomic DNA, genomic DNA library, cDNA derived from the cells and tissues described above, cDNA library derived from the cells and tissues described above and synthetic DNA.

The DNA encoding the partial peptide of the present invention is exemplified by DNA having a part of the base sequence represented by SEQ ID NO: 2 or DNA having a base sequence hybridizable to DNA having the base sequence represented by SEQ ID NO: 2 under high stringent conditions and having a part of DNA encoding a protein having a substantially equivalent activity as that of the protein of the present invention.

The DNA hybridizable to DNA having the base sequence represented by SEQ ID NO: 2 has a same sense as described above.

Means for cloning DNA encoding the protein or the partial peptide of the present invention (hereafter, these may be referred to the protein of the present invention) are, for example, amplifying by employing PCR method using a synthesized DNA primer having a partial base sequence of the protein of the present invention, or selecting the DNA, which has been integrated in a proper vector, through hybridization with that labeled by using a DNA fragment or a synthesized DNA encoding partially or totally regions of the protein of the present invention.

The hybridization can be carried out by publicly known methods or by modifications of these methods, for example, according to the method described in Molecular Cloning, 2nd Ed.; J. Sambrook et al., Cold Spring Harbor Lab. Press, (1989). A commercially available library may also be used according to the instructions of the attached manufacturer's protocol. The hybridization can be carried out preferably under high stringent conditions.

Substitution of the base sequence of DNA can be carried out according to publicly known methods such as the Gapped duplex method, the Kunkel method, etc. or their modifications by using publicly known kits available as Mutan™-G (Takara Shuzo Co., Ltd.), Mutan™-K (Takara Shuzo Co., Ltd.), etc.

The DNA encoding the cloned protein can be used as it is, depending upon purpose or, if desired, after digestion with a restriction enzyme or after addition of a linker thereto. The DNA may contain ATG as a translation initiation codon at the 5' end thereof and TM, TGA or TAG as a translation termination codon at the 3' end thereof. These translation initiation and termination codons may also be added by using an appropriate synthetic DNA adapter.

The expression vector of the protein of the present invention can be manufactured, for example, by (a) excising the desired DNA fragment from the DNA, e.g., cDNA, encoding the protein of the present invention, (b) followed by ligating the DNA fragment with an appropriate expression vector downstream a promoter in the vector.

Examples of the vector which can be used include plasmids derived form E. coli (e.g., pBR322, pBR325, pUC12, pUC13), plasmids derived from Bacillus subtilis (e.g., pUB110, pTP5, pC194), plasmids derived from yeast (e.g., pSH19, pSH15), bacteriophages such as λ phage, etc., animal viruses such as retrovirus, vaccinia virus, baculovirus, etc. as well as pA1-11, pXT1, pRc/CMV, pRc/RSV, pcDNAl/Neo, etc.

The promoter used in the present invention may be any promoter if it matches well with a host to be used for gene expression. In the case of using animal cells as the host, examples of the promoter include SRα promoter, SV40 promoter, LTR promoter, CMV promoter, HSV-TK promoter, etc.

Among them, CMV promoter or SRα promoter is preferably used. When the host is bacteria of the genus Escherichia, preferred examples of the promoter include trp promoter, lac promoter, recA promoter, λP_{L} promoter, Ipp promoter, etc. In the case of using bacteria of the genus Bacillus as the host, preferred example of the promoter are SPO1 promoter, SPO2 promoter and penP promoter. In the case of using yeast as the host, preferred examples of the promoter are PHO5 promoter, PGK promoter, GAP promoter and ADH promoter. In the case of using insect cells as the host, preferred examples of the promoter include polyhedrin promoter, P10 promoter, and the like.

In addition to the foregoing examples, the expression vector may further optionally contain an enhancer, a splicing signal, a polyA addition signal, a selection marker, SV40 replication origin (hereafter sometimes abbreviated as SV40ori) etc. Examples of the selection marker include dihydrofolate reductase (hereafter sometimes abbreviated as dhfr) gene [methotrexate (MTX) resistance], ampicillin resistant gene (hereafter sometimes abbreviated as Amp^{r}), neomycin resistant gene (hereafter sometimes abbreviated as Neo^{r}, G418 resistance), etc. In particular, when the dhfr gene is used as the selection marker using a dhfr gene-lack Chinese hamster cell, selection of the target gene can also be made by using a thymidine free medium.

If necessary, DNA encoding a signal sequence that matches with a host is added to the 5' terminus of the DNA encoding the protein of the present invention. Examples of the signal sequence that can be used are Pho A signal sequence, OmpA signal sequence, etc. in the case of using bacteria of the genus Escherichia as the host; α-amylase signal sequence, subtilisin signal sequence, etc. in the case of using bacteria of the genus Bacillus as the host; MFαsignal sequence, SUC2 signal sequence, etc. in the case of using yeast as the host; and insulin signal sequence, α-interferon signal sequence, antibody molecule signal sequence, etc. in the case of using animal cells as the host, respectively.

Using the vector containing the DNA encoding the protein of the present invention thus constructed, transformants can be manufactured.

Examples of the host, which may be employed, are bacteria belonging to the genus Escherichia, bacteria belonging to the genus Bacillus, yeast, insect cells, insects and animal cells, etc.

Specific examples of bacteria belonging to the genus Escherichia include Escherichia coli K12 DH1 [Proc. Natl. Acad. Sci. U.S.A., 60, 160 (1968)), JM103 (Nucleic Acids Research, 9, 309 (1981)), JA221 (Journal of Molecular Biology, 120, 517 (1978)], HB101 [Journal of Molecular Biology, 41, 459 (1969)), C600 (Genetics, 39, 440 (1954)], etc.

Examples of bacteria belonging to the genus Bacillus include Bacillus subtilis MI114 [Gene, 24, 255 (1983)], 207-21 [Journal of Biochemistry, 95, 87 (1984)], etc.

Examples of yeast include Saccharomyces cereviseae AH22, AH22R⁻, NA87-11A, DKD-5D, 20B-12, Schizosaccharomyces pombe NCYC1913, NCYC2036, Pichia pastoris KM71, etc.

Examples of insect cells include, for the virus AcNPV, Spodoptera frugiperda cell (Sf cell), MG1 cell derived from mid-intestine of Trichoplusia ni, High Five™ cell derived from egg of Trichoplusia ni, cells derived from Mamestra brassicae, cells derived from Estigmena acrea, etc.; and for the virus BmNPV, Bombyx mori N cell (BmN cell), etc. Examples of the Sf cell which can be used are Sf9 cell (ATCC CRL1711), Sf21 cell (both cells are described in Vaughn, J. L. et al., In Vivo, 13, 213-217 (1977), etc.

As the insect, for example, a larva of Bombyx mori can be used [Maeda et al., Nature, 315, 592 (1985)].

Examples of animal cells include monkey cell COS-7, Vero, Chinese hamster cell CHO (hereafter referred to as CHO cell), dhfr gene deficient Chinese hamster cell CHO (hereafter simply referred to as CHO(dhfr⁻) cell), mouse L cell, mouse AtT-20, mouse myeloma cell, rat GH 3, human FL cell, etc.

Bacteria belonging to the genus Escherichia can be transformed, for example, according to the method described in Proc. Natl. Acad. Sci. U.S.A., 69, 2110 (1972) or Gene, 17, 107 (1982), etc.

Bacteria belonging to the genus Bacillus can be transformed, for example, according to the method described in Molecular & General Genetics, 168, 111 (1979), etc.

Yeast can be transformed, for example, according to the method described in Methods in Enzymology, 194, 182-187 (1991) or Proc. Natl. Acad. Sci. U.S.A., 75, 1929 (1978), etc.

Insect cells or insects can be transformed, for example, according to the method described in Bio/Technology, 6, 47-55(1988), etc.

Animal cells can be transformed, for example, according to the method described in Saibo Kogaku (Cell Engineering), extra issue 8, Shin Saibo Kogaku Jikken Protocol (New Cell Engineering Experimental Protocol), 263-267 (1995), published by Shujunsha, or Virology, 52, 456 (1973).

Thus, the transformant transformed with the expression vector containing the DNA encoding the protein of the present invention can be obtained.

Where the host is bacteria belonging to the genus Escherichia or the genus Bacillus, the transformant can be appropriately incubated in a liquid medium which contains materials required for growth of the transformant such as carbon sources, nitrogen sources, inorganic materials, etc. Examples of the carbon sources include glucose, dextrin, soluble starch, sucrose, etc. Examples of the nitrogen sources include inorganic or organic materials such as ammonium salts, nitrate salts, corn steep liquor, peptone, casein, meat extract, soybean cake, potato extract, etc. Examples of the inorganic materials are calcium chloride, sodium dihydrogenphosphate, magnesium chloride, etc. In addition, yeast, vitamins, growth promoting factors etc. may also be added to the medium. Preferably, pH of the medium is adjusted to about 5 to about 8.

A preferred example of the medium for incubation of the bacteria belonging to the genus Escherichia is M9 medium supplemented with glucose and Casamino acids (Miller, Journal of Experiments in Molecular Genetics, 431-433, Cold Spring Harbor Laboratory, New York, 1972). If necessary, a chemical such as 3β-indolylacrylic acid can be added to the medium thereby to activate the promoter efficiently.

Where the bacteria belonging to the genus Escherichia are used as the host, the transformant is usually cultivated at about 15 to about 43°C for about 3 hours to about 24 hours. If necessary, the culture may be aerated or agitated.

Where the bacteria belonging to the genus Bacillus are used as the host, the transformant is cultivated generally at about 30 to about 40°C for about 6 hours to about 24 hours. If necessary, the culture can be aerated or agitated.

Where yeast is used as the host, the transformant is cultivated in, for example, Burkholder's minimal medium [Bostian, K. L. et al., Proc. Natl. Acad. Sci. U.S.A., 77, 4505 (1980)] or in SD medium supplemented with 0.5% Casamino acids [Bitter, G. A. et al., Proc. Natl. Acad. Sci. U.S.A., 81, 5330 (1984)]. Preferably, pH of the medium is adjusted to about 5 to about 8. In general, the transformant is cultivated at about 20°C to about 35°C for about 24 hours to about 72 hours. If necessary, the culture can be aerated or agitated.

Where insect cells or insects are used as the host, the transformant is cultivated in, for example, Grace's Insect Medium [Grace, T. C. C., Nature, 195, 788 (1962)] to which an appropriate additive such as fixed 10% bovine serum is added. Preferably, pH of the medium is adjusted to about 6.2 to about 6.4. Normally, the transformant is cultivated at about 27°C for about 3 days to about 5 days and, if necessary, the culture can be aerated or agitated.

Where animal cells are employed as the host, the transformant is cultivated in, for example, MEM medium containing about 5% to about 20% fetal bovine serum (Science, 122, 501 (1952)], DMEM medium [Virology, 8, 396 (1959)), RPMI 1640 medium [The Journal of the American Medical Association, 199, 519 (1967)], 199 medium [Proceeding of the Society for the Biological Medicine, 73, 1 (1950)], etc. Preferably, pH of the medium is adjusted to about 6 to about 8. The transformant is usually cultivated at about 30°C to about 40°C for about 15 hours to about 60 hours and, if necessary, the culture can be aerated or agitated.

As described above, the protein of the present invention can be produced in the cells or cell membranes of the transformants, or outside the transformants.

The protein of the present invention can be separated and purified from the culture described above by the following procedures.

When the protein of the present invention is extracted from the culture or cells, after cultivation, the transformants or cells is collected by publicly known methods and suspended in an appropriate buffer. The transformants or cells are then disrupted by publicly known methods such as ultrasonication, a treatment with lysozyme and/or freeze-thaw cycling, etc., followed by centrifugation, filtration, etc. Thus, the crude extract of the protein can be obtained. The buffer used for the procedures may contain a protein modifier such as urea or guanidine hydrochloride, or a surfactant such as Triton X-100™, etc. When the protein of the present invention is secreted in the culture broth, after completion of the cultivation the supernatant can be separated from the transformants or cells to collect the supernatant by publicly known methods.

The protein of the present invention contained in the supernatant or extract thus obtained can be purified by appropriately combining the publicly known methods for separation and purification. Such publicly known methods for separation and purification include a method utilizing difference in solubility such as salting out, solvent precipitation, etc.; a method mainly utilizing difference in molecular weight such as dialysis, ultrafiltration, gel filtration, SDS-polyacrylamide gel electrophoresis, etc.; a method utilizing difference in electric charge such as ion exchange chromatography, etc.; a method utilizing difference in specific affinity such as affinity chromatography, etc.; a method utilizing difference in hydrophobicity such as reverse phase high performance liquid chromatography, etc.; a method utilizing difference in isoelectric point such as isoelectrofocusing electrophoresis; and the like.

When the protein of the present invention thus obtained is in a free form, it can be converted into the salt by publicly known methods or modifications thereof. On the other hand, when the protein is obtained in the form of a salt, it can be converted into the free form or in the form of a different salt by publicly known methods or modifications thereof.

The protein produced by the recombinant can be treated, prior to or after the purification, with an appropriate protein-modifying enzyme so that the protein can be appropriately modified to partially remove a polypeptide. Examples of the protein-modifying enzyme include trypsin, chymotrypsin, arginyl endopeptidase, protein kinase, glycosidase and the like.

The presence of the thus produced protein of the present invention can be measured by enzyme immunoassay or western blotting using a specific antibody.

The antibody to the protein of the present invention or its partial peptide, or salts thereof may be any of polyclonal and monoclonal antibodies, so long as they can recognize the protein of the present invention or salts thereof.

The antibody to the protein of the present invention or its peptide, or salts thereof (hereafter sometimes merely referred to as the protein of the present invention) can be manufactured by publicly known methods for manufacturing antibodies or antisera, using the protein, as an antigen, of the present invention.

### [Preparation of monoclonal antibody]

### (a) Preparation of monoclonal antibody-producing cells

The protein or the like of the present invention is administered to a warm-blooded animal, either solely or together with carriers or diluents to the site that can produce the antibody by the administration. In order to potentiate the antibody productivity upon the administration, complete Freund's adjuvant or incomplete Freund's adjuvant may be administered. The administration is effected usually once every 2 to 6 weeks and approximately 2 to 10 times in total. The warm-blooded animals to be used include monkey, rabbit, dog, guinea pig, mouse, rat, sheep, goat, and fowl with mouse and rat being preferred.

In the preparation of the monoclonal antibody-producing cells, an animal wherein the antibody titer is noted is selected from warm-blooded animals immunized with antigens, e.g., mice, then spleen or lymph node is collected after two to five days from the final immunization and the antibody-producing cells contained therein are fused with myeloma cells derived from an animal of a same or a different species to give monoclonal antibody-producing hybridomas. The antibody titer in antisera may be determined, for example, by reacting with a labeled protein, which will be described later, with the antiserum followed by measuring the binding activity of the labeling agent bound to the antibody. The fusion may be carried out, for example, according to the method of Koehler and Milstein (Nature, 256, 495, 1975). Examples of the fusion accelerator are polyethylene glycol (PEG), Sendai virus, etc. and PEG is preferably used.

Examples of myeloma cells include NS-1, P3U1, SP2/0, etc., with P3U1 being preferred. The ratio of the number of the antibody-producing cells (spleen cells) to the number of myeloma cells to be used is preferably about 1:1 to about 20:1 and PEG (preferably PEG 1000 to PEG 6000) is added in a concentration of about 10% to about 80%. The cell fusion can be efficiently carried out by incubating both cells at about 20°C to about 40°C, preferably about 30°C to about 37°C for about 1 minute to about 10 minutes.

Various methods can be used for screening of a monoclonal antibody-producing hybridoma. Examples of such methods include a method which comprises adding the supernatant of hybridoma to a solid phase (e.g., microplate) adsorbed with the protein as antigen directly or together with a carrier, adding an anti-immunoglobulin antibody (where mouse cells are used for the cell fusion, anti-mouse immunoglobulin antibody is used) labeled with a radioactive substance or an enzyme or Protein A and detecting the monoclonal antibody bound to the solid phase, and a method which comprises adding the supernatant of hybridoma to a solid phase adsorbed with an anti-immunoglobulin antibody or Protein A, adding the protein labeled with a radioactive substance or an enzyme and detecting the monoclonal antibody bound to the solid phase.

The monoclonal antibody can be selected according to publicly known methods or their modifications. In general, the selection can be effected in a medium for animal cells supplemented with HAT (hypoxanthine, aminopterin and thymidine). Any selection and growth medium can be employed as far as the hybridoma can grow therein. For example, RPMI 1640 medium containing 1% to 20%, preferably 10% to 20% fetal bovine serum, GIT medium (Wako Pure Chemical Industries, Ltd.) containing 1% to 10% fetal bovine serum, a serum free medium for cultivation of a hybridoma (SFM-101, Nissui Seiyaku Co., Ltd.) and the like can be used for the selection and growth medium. The cultivation is carried out generally at 20°C to 40°C, preferably 37°C, for about 5 days to about 3 weeks, preferably 1 to 2 weeks, normally in 5% CO₂. The antibody titer of the culture supematant of a hybridoma can be determined as in the determination of antibody titer in antisera described above.

### (b) Purification of monoclonal antibody

Separation and purification of a monoclonal antibody can be carried out according the same manner as applied to the publicly known method, such as separation and purification of immunoglobulins [for example, salting-out, alcohol precipitation, isoelectric point precipitation, electrophoresis, adsorption and desorption with ion exchangers (e.g., DEAE), ultracentrifugation, gel filtration, or a specific purification method which comprises collecting only an antibody with an activated adsorbent such as an antigen-binding solid phase, Protein A or Protein G and dissociating the binding to obtain the antibody].

### [Preparation of polyclonal antibody]

The polyclonal antibody of the present invention can be manufactured by publicly known methods or modifications thereof. For example, an immunogen (the protein as an antigen) itself or its complex with a carrier protein is formed and a warm-blooded animal is immunized with the complex in a manner similar to the method described above for the manufacture of monoclonal antibody. The product containing the antibody to the protein or the like of the present invention is collected from the immunized animal followed by separation and purification of the antibody.

In the complex of immunogen and carrier protein for immunizing a warm-blooded animal, the type of carrier protein and the mixing ratio of carrier to hapten may be any type and in any ratio, as long as the antibody is efficiently produced to the hapten immunized by crosslinking to the carrier. For example, bovine serum albumin, bovine thyroglobulin or hemocyanin is coupled to hapten in a carrier-to-hapten weight ratio of approximately 0.1 to 20, preferably 1 to 5.

A variety of condensation agents can be used for the coupling of carrier to hapten. Glutaraldehyde, carbodiimide, maleimide activated ester and activated ester reagents containing thiol group or dithiopyridyl group are used for the coupling.

The condensation product is administered to warm-blooded animals either solely or together with carriers or diluents to the site that can produce the antibody by the administration. In order to potentiate the antibody productivity upon the administration, complete Freund's adjuvant or incomplete Freund's adjuvant may be administered. The administration is usually carried out once approximately every 2 to 6 weeks and about 3 to about 10 times in total.

The polyclonal antibody can be collected from the blood, ascites, etc., preferably from the blood of warm-blooded animals immunized by the method described above.

The polyclonal antibody titer in antiserum can be determined by the same procedure as in the serum antibody titer described above. The polyclonal antibody can be separated and purified according to the same method for separation and purification of immunoglobulin as used for the monoclonal antibody described above.

An antisense DNA having a complementary or substantially complementary base sequence to DNA encoding the protein or the partial peptide of the present invention (hereafter, these DNA may be referred to the DNA of the present invention) may be any antisense DNA having a complementary or substantially complementary base sequence to DNA of the present invention and having an action to suppress the expression of the DNA.

The substantially complementary base sequence to the DNA of the present invention is exemplified by the base sequence having about 70% or higher, preferably about 80% or higher, more preferably about 90% or higher, most preferably about 95% or higher homology to all the base sequences or a partial base sequence of the base sequence complementary to the DNA of the present invention (i.e., a complementary chain of the DNA of the present invention). Particularly, among all the base sequence of the complementary chain of the DNA of the present invention, a preferable antisense DNA is those having about 70% or higher, preferably about 80% or higher, more preferably about 90% or higher, most preferably about 95% or higher homology to the complementary chain of the base sequence (e. g., the base sequence around an initiation codon) of a part encoding the N-terminal domain of the protein of the present invention. These antisense DNAs can be manufactured by using a publicly known DNA synthesizer.

Hereafter the use of the protein of the invention or its partial peptide, or salts thereof (hereafter sometimes collectively referred to as the protein, etc. of the invention); the DNA encoding the protein of the invention or its partial peptide (hereafter sometimes collectively referred to as the DNA of the invention), and the antibody to the protein of the invention or its partial peptide, or salts thereof (hereafter sometimes collectively referred to as the antibody of the invention) and an antisense DNA.

### (1) A prophylactic and/or therapeutic agent for various diseases with which the protein of the invention is associated

The protein of the invention is bound to IRAP to fix intracellularly the GLUT4 vesicle (vesicle where such proteins as GLUT4, IRAP, VAMPs, SCAMPs, Rab4, and the like are locally present) and inhibit intake of blood glucose in a muscular cell and a fat cell to raise the blood glucose level.

Thus, the protein of the invention and the DNA of the invention can be used as pharmaceuticals such as prophylactic and therapeutic agents for various diseases hypoglycemia, etc.

When a patient has a reduced level of, or deficient in the protein of the invention in his or her body causing an insufficient and improper expression of homeostasis or biophylactic mechanisms of the living body, a role of the protein of the present invention can provide sufficiently or properly for the patient, (a) by administering the DNA of the invention to the patient to express the protein, etc, of the invention in vivo, (b) by inserting the DNA of the invention into a cell, expressing the protein, etc. of the invention and then transplanting the cell to the patient, or (c) by administering the protein, etc. of the invention to the patient.

Where the DNA of the invention is used as the prophylactic/therapeutic agents described above, the DNA is administered directly to human or other warm-blooded animal; alternatively, the DNA is inserted into an appropriate vector such as retrovirus vector, adenovirus vector, adenovirus-associated virus vector, etc. and then administered to human or other warm-blooded animal in a conventional manner. The DNA of the invention may also be administered as naked DNA, or with physiologically acceptable carrier such as adjuvants to assist its uptake by gene gun or through a catheter such as a catheter with a hydrogel.

Where the protein of the invention is used as the aforesaid therapeutic/prophylactic agents, the protein is advantageously used on a purified level of at least 90%, preferably at least 95%, more preferably at least 98% and most preferably at least 99%.

The protein of the invention can be used orally, for example, in the form of tablets which may be sugar coated if necessary, capsules, elixirs, microcapsules etc., in a form of inhalant prepared by making in an aerosol product, or parenterally in the form of injectable preparations such as a sterile solution and a suspension in water or with other pharmaceutically acceptable liquid. These preparations can be manufactured by mixing the protein, etc. of the invention with a physiologically acceptable carrier, a flavoring agent, an excipient, a vehicle, an antiseptic agent, a stabilizer, a binder, etc. in a unit dosage form required in a generally accepted manner that is applied to making pharmaceutical preparations. The active ingredient in the preparation is controlled in such a dose that an appropriate dose is obtained within the specified range given.

Additives miscible with tablets, capsules, etc. include a binder such as gelatin, corn starch, tragacanth and gum arabic, an excipient such as crystalline cellulose, a swelling agent such as corn starch, gelatin and alginic acid, a lubricant such as magnesium stearate, a sweetening agent such as sucrose, lactose and saccharin, and a flavoring agent such as peppermint, akamono oil and cherry. When the unit dosage is in the form of capsules, liquid carriers such as oils and fats may further be used together with the additives described above. A sterile composition for injection may be formulated according to a conventional manner used to make pharmaceutical compositions, e.g., by dissolving or suspending the active ingredients in a vehicle such as water for injection with a naturally occurring vegetable oil such as sesame oil and coconut oil, etc. to prepare the pharmaceutical composition.

Examples of an aqueous medium for injection include physiological saline and an isotonic solution containing glucose and other auxiliary agents (e.g., D-sorbitol, D-mannitol, sodium chloride, etc.) and may be used in combination with an appropriate dissolution aid such as an alcohol (e.g., ethanol or the like), a polyalcohol (e.g., propylene glycol and polyethylene glycol), a nonionic surfactant (e.g., polysorbate 80TM, HCO-50, etc.), or the like. Examples of the oily medium include sesame oil and soybean oil, which may also be used in combination with a dissolution aid such as benzyl benzoate and benzyl alcohol. The liquid for injection described above may further be formulated with a buffer (e.g., phosphate buffer, sodium acetate buffer, etc.), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, etc. The thus prepared liquid for injection is normally filled in an appropriate ampoule.

The vector in which the DNA of the invention is inserted may also be prepared into pharmaceutical preparations in a manner similar to the procedures above. Such preparations are generally used parenterally.

Since the thus obtained pharmaceutical preparation is safe and low toxic, the preparation can be administered to a human and other warm-blooded animals (e.g., rat, mouse, guinea pig, rabbit, bird, sheep, swine, bovine, horse, cat, dog, monkey, chimpanzee, etc.).

A dose of the protein of the invention varies depending on target disease, subject to be administered, for administration, etc.; when the protein, etc. of the invention is orally administered for the purpose of treatment for, e.g., hypoglycemia, the protein, etc. is administered to adult (as 60 kg body weight) normally in a daily dose of about 0.1 mg to about 100 mg, preferably about 1.0 to about 50 mg, and more preferably about 1.0 to about 20 mg. In parenteral administration, a single dose of the protein varies depending on subject to be administered, target disease, etc. but when the protein, etc. of the invention is administered to adult (as 60 kg body weight) in the form of injection for the purpose of treatment for hypoglycemia, it is advantageous to administer the protein in a daily dose of about 0.01 to 30 mg, preferably about 0.1 to 20 mg, and more preferably about 0.1 to 10 mg. For other animal species, the corresponding dose as converted per 60 kg body weight can be administered.

### (2) Screening of a binding-inhibition substance

The protein of the invention is bound to a cytoplasm side domain of IRAP to fix intracellularly the GLUT4 vesicle resulting in inhibiting intake of blood glucose in a muscular cell and a fat cell. Hence, a compound inhibiting binding of the protein of the invention to IRAP, preferably the compound inhibiting binding of the protein of the invention to the cytoplasm side domain of IRAP, can promote intake of blood glucose in the muscular cell and the fat cell to lower blood glucose and is useful as a prophylactic and therapeutic agent of, for example, hyperglycemia, diabetes, (type 1 diabetes, type 2 diabetes, pregnancy diabetes,) impaired glucose tolerance (IGT,) diabetic complication (e.g., diphtheritic neuropathy, nephropathy, retinopathy, cataract, great vessel injury, bone reduction, diabetic hyperosmolar coma, infectious disease (e.g., respiratory infection, infectiousness of genito-urinary tract, infectiousness of digestive tract, infectiousness of soft part tissue of skin, infectiousness of lower limb, et,,) diabetic gangraena, dry mouth, auditory degradation, cerebral vascular accident, peripheral circulatory disturbance, etc.,) and pharmaceuticals such as an agent suppressing to change from impaired glucose tolerance to diabetes.

For a decision standard of diabetes, a new decision standard was published from Japan Diabetes Association in 1999.

According to this publication, diabetes is defined as a status showing any one of that a fasting blood glucose value (glucose concentration in a vein plasma) is 126 mg/dl or higher, that 2-hour value (glucose concentration in a vein plasma) of 75 g oral glucose tolerance test (75 g OGTT) is 200 mg/dl or higher, and that casual blood glucose value (glucose concentration in a vein plasma) is 200 mg/dl or higher. On the other hand, the status applicable to diabetes as described above and is not " a status in which the fasting blood glucose value (glucose concentration in a vein plasma) is less than 110 mg/dl and the 2-hour value (glucose concentration in a vein plasma) of 75 g oral glucose tolerance test (75 g OGTT) is less than 140 mg/dl" (normal type) is called "borderline type."

For the decision standard of diabetes, the new decision standard was published from ADA (American Diabetes Association) in 1997 and from WHO in 1998.

According to these publications, diabetes is defined as the status showing that the fasting blood glucose value (glucose concentration in a vein plasma) is 126 mg/dl or higher and the 2-hour value (glucose concentration in a vein plasma) of 75 g oral glucose tolerance test is 200 mg/dl or higher.

Also according to these publications, impaired glucose tolerance is defined as the status showing that the fasting blood glucose value (glucose concentration in a vein plasma) is less than 126 mg/dl and the 2-hour value (glucose concentration in a vein plasma) of 75 g oral glucose tolerance test is 140 mg/dl or higher and less than 200 mg/dl. Moreover, according to the ADA report, the status, in which the fasting blood glucose value (glucose concentration in a vein plasma) is 110 mg/dl or higher and less than 126 mg/dl, called IFG (Impaired Fasting Glucose.) On the other hand, according to the WHO report, concerning the IFG (Impaired Fasting Glucose) values, the status, in which the 2-hour value (glucose concentration in a vein plasma) of 75 g oral glucose tolerance test is less than 140 mg/dl, is called IFG (Impaired Fasting Glycemia.)

The compound inhibiting binding of the protein of the present invention to IRAP is used as the prophylactic and therapeutic agent of diabetes, borderline type, impaired glucose tolerance, the IFG (Impaired Fasting Glucose,) and the IFG (Impaired Fasting Glycemia) that are determined on the basis of the new decision standard as described above.

The compound inhibiting binding of the protein of the present invention to IRAP can prevent to develop from the IFG (Impaired Fasting Glucose,) and the IFG (Impaired Fasting Glycemia) to diabetes.

The compound inhibiting binding of the protein of the present invention to IRAP is exemplified by the compounds expressed by the following formulae:

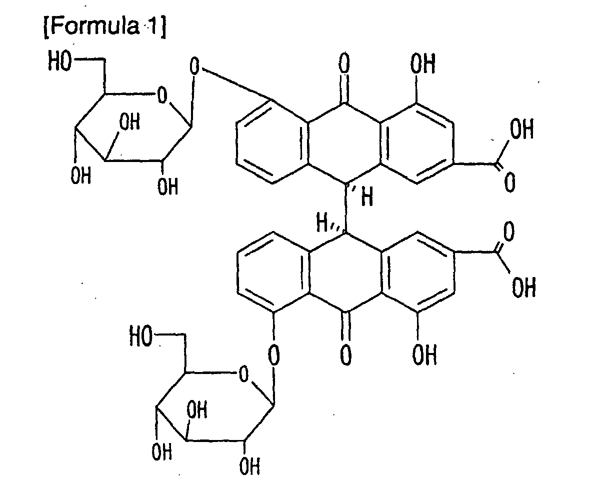

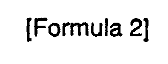

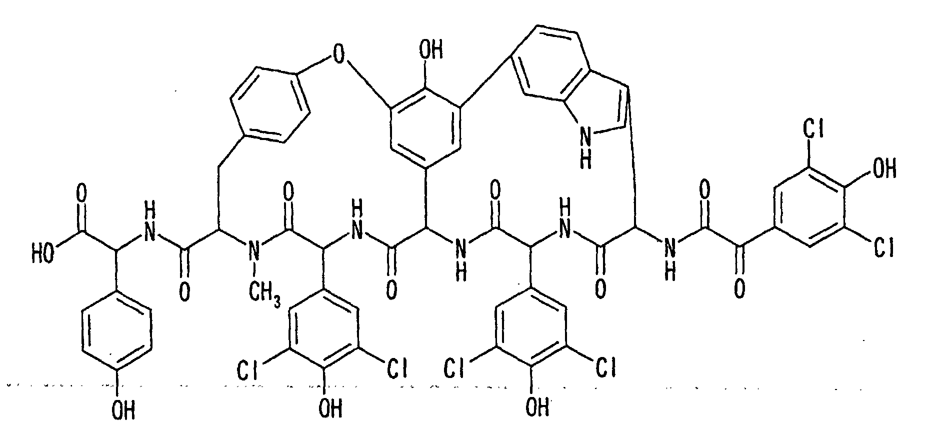

The protein of the invention is also bound to a cytoplasm side domain of GLUT4 (amino acid numbers 468 to 510 of GLUT4; SEQ ID NO: 7) to fix intracellularly the GLUT4 vesicle resulting in inhibiting intake of blood glucose in the muscular cell and the fat cell. Hence, the compound inhibiting binding of the protein of the invention to GLUT4, preferably the compound, as similar to the compound inhibiting binding of the protein of the invention to IRAP, inhibiting binding of the protein of the invention to the cytoplasm side domain of GLUT4 is useful as the prophylactic and therapeutic agent of, for example, hyperglycemia and diabetes.

For the screening method of the invention, the protein of the invention may be used and the peptide corresponding to IRAP or the domain of IRAP in the cytoplasm side or the peptide corresponding to GLUT4 or the domain of GLUT4 in the cytoplasm may be used. For the screening method of the invention, the cell (preferably, the transformant (cells such as yeast, animal cell, etc.) transformed by the DNA encoding the protein of the invention) having ability of producing the protein of the invention may be used. The transformant may be the transformant transformed by the DNA encoding the protein of the invention and the DNA encoding the peptide corresponding to IRAP or the domain of IRAP in the cytoplasm side, or the transformant transformed by the DNA encoding the protein of the invention and the DNA encoding the peptide corresponding to GLUT4 or the domain of GLUT4 in the cytoplasm.

### (2-1) Screening by in vitro binding test

The protein of the invention is fixed to the solid phase (EIA plate) by using an antibody against the protein of the invention or fixed to the solid phase after fusing the protein of the invention with a Tag protein (e.g., His-Tag, GST (glutathione-S-transferase,) etc.) In the case where the partial peptide of the present invention is used as the protein of the invention, the partial peptide (amino acid numbers from 36 to 655 of SEQ ID NO: 1) having the binding activity to IRAP or GLUT4 is preferably used. In fixing the protein to the solid phase, nickel and glutathione are used for His-Tag and GST, respectively. To this, the partial peptide (amino acid sequence or its partial sequence represented by SEQ ID NO: 5; preferably amino acid sequence from 55 to 82 of SEQ ID NO: 5) corresponding to IRAP or the domain of IRAP in the cytoplasm side or the partial peptide (SEQ ID NO: 7) corresponding to GLUT4 or the domain of GLUT4 in the cytoplasm is labeled with biotin and added for coupling. A test compound is added to this complex, and IRAP or the IRAP partial peptide or GLUT4 or the GLUT4 partial peptide, which has become free as the result of inhibition of coupling of the protein of the invention to IRAP or GLUT4, is detected to quantify using a commercial kit for detection of a labeled body such as biotin or a publicly known anti-IRAP antibody or a commercial anti- GLUT4 antibody. The compound making free IRAP or the IRAP partial peptide or GLUT4 or the GLUT4 partial peptide is selected as the compound (hereafter may be denoted as a binding inhibitor) to inhibit a coupling of the protein of the invention to IRAP or GLUT4.

The partial peptide (amino acid sequence or its partial sequence represented by SEQ ID NO: 5; preferably amino acid sequence from 55 to 82 of SEQ ID NO: 5) corresponding to IRAP or the domain of IRAP in the cytoplasm side or the partial peptide (SEQ ID NO: 7) corresponding to GLUT4 or the domain of GLUT4 in the cytoplasm is fixed to the solid phase to bind to the protein of the invention. When IRAP or the IRAP partial peptide or GLUT4 or the GLUT4 partial peptide is fixed to the solid phase, for example, IRAP or the IRAP partial peptide or GLUT4 or the GLUT4 partial peptide, which is labeled with biotin, and the solid phase (e.g., a plate) labeled with avidin are preferably used. The test compound is added to this complex and the protein of the invention liberated is detected and quantified using the antibody against the protein of the invention or the antibody against the tag protein. As the protein of the invention for this method, the protein of the invention may be used after fusion with the tag protein. In this way, the protein of the invention liberated may be detected and quantified using the antibody against the protein of the invention or may be detected and quantified using the antibody against the tag protein. The compound liberating the protein of the invention is selected as the binding inhibitor.

Inhibition activity of the compound selected can be confirmed by the publicly known method such as immune precipitation using an anti-IRAP antibody, an anti-GLUT4 antibody, the antibody against the protein of the invention, or the antibody against Tag. In the immune precipitation, the protein of the invention or IRAP or GLUT4, that is liberated through inhibition of the bond of the protein of the invention to IRAP or GLUT4 by the selected compound, is detected by the antibody against the protein of the invention, the antibody against the tag protein, the antibody against IRP, or the antibody against GLUT4.

### (2-2) Screening by two-hybrid method.

### (2-2-1) Screening by enzyme two-hybrid method.

In yeast (Saccharomyces cerevisiae, more preferably S. cerevisiae Y190,) when the DNA encoding the partial peptide corresponding to the domain of IRAP, which has fused with a reporter gene-binding domain, in the cytoplasm side or the partial peptide corresponding to the above-described domain of GLUT4 in the cytoplasm and the DNA encoding the protein of the invention fused with a reporter gene transcription-active domain are expressed, the two-hybrid works to express a phenotype of β-galactosidase being the reporter gene and the histidine synthesis gene HIS3. This yeast strain is cultured for a certain time period in the presence of the test compound to reduce β-galactosidase activity of the yeast strain or to select the compound to convert the yeast strain into a histidine-requiring strain. The yeast strain can be cultured in the same way as that of the transformant of which host as describe above is yeast. The activity of β-galactosidase can be measured after the publicly known method using X-Gal (5-bromo-4-chloro-3-inddolyl-β-D-galactopyranoside,) ONPG (o-nitrophenyl β-D-galactopyranoside,) or CPRG (chlorophenyl red-β-D-galactopyranoside) as the substrate. Expression of HIS3 can be measured by culturing yeast using the minimum essential medium lacking histidine. Among the selected compounds, the compound showing cytotoxicity and the compound inhibiting activity of a reporter gene product itself by an interaction with the reporter gene product can be removed as pseudopositive compounds.

### (2-2-2) Screening by two-hybrid method using animal cells.

The reporter genes such as chloramphenicol acetyltransferase (CAT) gene or firefly luciferase gene are transduced in an animal cell (e.g., Chinese hamster ovary (CHO) cell.) A transcriptional control region of the reporter gene used is that prepared by combining, e.g., a transcription active sequence (UAS) of GAL1, with a downstream of the promoter (e.g., a minimum promoter (TATA box) or the like derived from adenovirus E1b) working in the animal cell. GAL4- GAL1 transcriptional control system is transduced in the animal cell of the enzyme two-hybrid system to allow expression of the reporter gene to induce in the animal cell. Expressing the DNA encoding the partial peptide corresponding to the above-described domain of IRAP, which has fused with a GAL4-DNA-binding domain, in the cytoplasm side or the partial peptide corresponding to the above-described domain of GLUT4 in the cytoplasm and the DNA encoding the protein of the invention, which has fused with the DNA encoding VP16 protein derived, e.g., from simple herpes virus, yields the animal cell line in which the reporter gene is expressed by the action of the two-hybrid system. This cell line is cultured for a certain time period in the presence of the test compound, the activity of the reporter gene product is measured, and the compound lowering the activity is selected. The animal cell line can be cultured in the same way as the culture of the transformant of which host as describe above is the animal cell. The activity of the reporter gene product can be measured after the publicly known method using the commercial kit. Among the selected compounds, the compound showing cytotoxicity and the compound inhibiting the activity of the reporter gene product itself by the interaction with the reporter gene product can be removed as pseudopositive compounds.

### (3) Screening of compounds promoting or suppressing expression of the protein of the invention.

The transcriptional control region of the DNA of the invention is cloned, this is fused with the reporter gene (e.g., β-galactosidase, firefly luciferase, chloramphenicol acetyltransferase (CAT,) etc.) to transduce in the animal cell (e.g., CHO cell.) This cell line is cultured for a certain time period in the presence of the test compound, and the compound increasing or lowering the amount of the reporter gene product is selected. The animal cell line can be cultured in the same way as the culture of the transformant of which host as describe above is the animal cell. Increasing or lowering of the amount of the reporter gene product can be determined, for example, by measuring the activity of the reporter gene product in the culture solution. Among the selected compounds, the compound showing cytotoxicity and the compound increasing or lowering the activity of the reporter gene product itself by the interaction with the reporter gene product can be removed as pseudopositive compounds.

Examples of the test compound include peptides, proteins, non-peptide compounds, synthetic compounds, fermentation products, cell extracts, plant extracts, animal tissue extracts, and the like and these compounds may be novel compounds or publicly known compounds. These compounds may be new compounds or publicly known compounds.

Examples of compounds suppressing expression of the protein of the invention include the compound, which is obtained by screening as described above, suppressing expression of the protein of the invention, the antisense DNA as described above, and the compound inhibiting the promoter activity to the DNA, described later, of the invention.

Examples of compounds promoting expression of the protein of the invention include the compound promoting expression of the protein of the invention, which is obtained by screening as described above, and the compound promoting the promoter activity to the DNA, described later, of the invention.

The kit for screening according to the invention includes the protein of the invention and may further include the peptide corresponding to IRAP or the domain of IRAP in the cytoplasm side or the peptide corresponding to GLUT4 or the domain of GLUT4 in the cytoplasm. The kit for screening according to the invention includes the transformant (e.g., cells such as yeast, animal cells, etc.) transformed by the DNA encoding the protein of the invention. The transformant may be the transformant transformed by the DNA encoding the protein of the invention and the DNA encoding the peptide corresponding to IRAP or the domain of IRAP in the cytoplasm side, or the transformant transformed by the DNA encoding the protein of the invention and the DNA encoding the peptide corresponding to GLUT4 or the domain of GLUT4 in the cytoplasm.

The compounds and salts thereof obtained by using the screening method or the screening kit of the invention are the test compounds as described above including peptides, proteins, non-peptide compounds, synthetic compounds, fermentation products, cell extracts, plant extracts, animal tissue extracts, blood plasma and the like and, for example, compounds are exemplified by the compound inhibiting the bond of the protein of the invention to IRAP or GLUT4, and the compound promoting or suppressing expression of the protein of the invention.

Examples of the salt of the compound used include those similar to the salt of the protein of the invention, as described above.

The compound inhibiting the bond of the protein of the invention to IRAP or GLUT4 or the compound suppressing expression of the protein of the invention are useful as the prophylactic and therapeutic agent of, for example, hyperglycemia and diabetes.

The compound promoting expression of the protein of the invention is useful as the prophylactic and therapeutic agent for diseases, for example, hypoglycemia, etc.

In the case where the compound or the salt thereof obtained by using the screening method or the screening kit of the invention is used as the prophylactic and therapeutic agent as described above, drug preparation can be carried out according to ordinary means. For example, the compounds can be administered orally or parenterally in the form of tablets, capsules, elixir, microcapsules, sterile solution, suspension, etc., in the same way as that of pharmaceuticals, as described above, containing the protein of the invention.

Since the thus obtained pharmaceutical preparation is safe and low toxic, the preparation can be administered to, for example, a human and other warm-blooded animals (e.g., mouse, rat, rabbit, sheep, swine, bovine, horse, fowl, cat, dog, monkey, chimpanzee, etc.).

A dose of the compound and the salt thereof varies depending on its action, a target disease, a subject to be administered, a route for administration, etc.; when the compound inhibiting the bond of the protein of the invention to IRAP or GLUT4 or the compound suppressing expression of the protein of the invention is orally administered for the purpose of treatment for, e.g., diabetes, the compound is administered to adult (as 60 kg body weight) normally in a daily dose of about 0.1 mg to about 100 mg, preferably about 1.0 to about 50 mg, and more preferably about 1.0 to about 20 mg. In parenteral administration, a single dose of the compound varies depending on the subject to be administered, the target disease, etc. but when the compound inhibiting the bond of the protein of the invention to IRAP or GLUT4 or the compound suppressing expression of the protein of the invention is administered to adult (as 60 kg body weight) in the form of injection for the purpose of treatment for, e.g., diabetes, it is advantageous to administer the compound intravenously in a daily dose of about 0.01 to 30 mg, preferably about 0.1 to 20 mg, and more preferably about 0.1 to 10 mg. For other animal species, the corresponding dose as converted per 60 kg body weight can be administered.

When the compound promoting expression of the protein of the invention is orally administered for the purpose of treatment for, e.g., hypoglycemia, the compound is administered to adult (as 60 kg body weight) normally in a daily dose of about 0.1 mg to about 100 mg, preferably about 1.0 to about 50 mg, and more preferably about 1.0 to about 20 mg. When the compound promoting expression of the protein of the invention is administered to adult (as 60 kg body weight) in the form of injection for the purpose of treatment for hypoglycemia, it is advantageous to administer the compound intravenously in a daily dose of about 0.01 to 30 mg, preferably about 0.1 to 20 mg, and more preferably about 0.1 to 10 mg. For other animal species, the corresponding dose as converted per 60 kg body weight.

### (4) Quantification of the protein of the present invention

The antibody against the protein of the present invention (hereafter, may be mentioned as the antibody of the present invention) is capable of specifically recognizing the protein of the present invention and accordingly, can be used for quantification of the protein of the present invention in a test sample, in particular, for quantification by sandwich immunoassay.

That is, the present invention provides
(i) a method of quantification of the protein of the present invention in a test sample, which comprises competitively reacting the antibody of the present invention, a test sample and a labeled form of the protein of the present invention, and measuring the ratio of the labeled protein of the present invention bound to the antibody; and,
(ii) a method of quantification of the protein of the present invention in a test sample, which comprises simultaneously or continuously reacting a test sample with the antibody of the present invention immobilized on an insoluble carrier and a labeled form of the antibody of the present invention, and measuring the activity of the labeling agent on the insoluble carrier.

In the method (ii) described above, it is preferred that one antibody is capable of recognizing the N-terminal region of the protein of the present invention, while another antibody is capable of recognizing the C-terminal region of the protein of the present invention.

The monoclonal antibody to the protein of the present invention (hereafter sometimes merely referred to as the monoclonal antibody of the present invention) may be used to assay the protein of the present invention. Moreover, the protein of the present invention can be detected by means of a tissue staining as well. For these purposes, the antibody molecule per se may be used or F(ab')₂, Fab' or Fab fractions of the antibody molecule may also be used.

There is no particular limitation for the quantification method using the antibody of the present invention against the protein of the present invention; any method may be used so far as it relates to a method in which the amount of antibody, antigen or antibody-antigen complex can be detected by a chemical or a physical means, depending on or corresponding to the amount of antigen (e.g., the amount of the protein) in a test sample to be assayed, and then calculated using a standard curve prepared by a standard solution containing the known amount of antigen. Advantageously used are, for example, nephrometry, competitive method, immunometric method and sandwich method; in terms of sensitivity and specificity, the sandwich method, which will be described later, is particularly preferred.

Examples of the labeling agents used in the assay methods using labeling substances are radioisotopes, enzymes, fluorescent substances, luminescent substances, etc. Examples of the radioisotope are [¹²⁵I], [¹³¹I], [³H], [¹⁴C], etc. Preferred examples of the enzyme are those that are stable and have a high specific activity, which include β-galactosidase, β-glucosidase, alkaline phosphatase, peroxidase and malate dehydrogenase. Examples of the fluorescent substance are fluorescamine, fluorescein isothiocyanate, etc. Examples of the luminescent substance are luminol, a luminol derivative, luciferin, lucigenin, etc. Furthermore, a biotin-avidin system may also be used for binding an antibody or antigen to a labeling agent.

In the insolubilization of antigens or antibodies, physical adsorption may be used. Alternatively, chemical binding that is conventionally used for insolubilization of proteins or enzymes may be used as well. Examples of the carrier include insoluble polysaccharides such as agarose, dextran and cellulose; synthetic resins such as polystyrene, polyacrylamide and silicone; glass; etc.

In the sandwich method, a test sample liquid is reacted with an immobilized monoclonal antibody of the present invention (primary reaction), then reacted with a labeled form of the monoclonal antibody of the present invention (secondary reaction) and the activity of the labeling agent on the insoluble carrier is assayed, whereby the amount of the protein of the present invention in the test sample liquid can be determined. The primary and secondary reactions may be carried out in a reversed order, simultaneously or sequentially with an interval. The type of the labeling agent and the method for immobilization may be the same as those described hereinabove. In the immunoassay by the sandwich method, it is not always necessary that the antibody used for the labeled antibody and for the solid phase should be one type or one species but a mixture of two or more antibodies may also be used for the purpose of improving the measurement sensitivity, etc.

In the method of assaying the protein by the sandwich method according to the present invention, the monoclonal antibodies of the present invention used for the primary and secondary reactions are preferably antibodies, which binding sites to the protein of the present invention are different from each other. Thus, the antibodies used in the primary and secondary reactions are those wherein, when the antibody used in the secondary reaction recognizes the C-terminal region of the protein of the present invention, the antibody recognizing the site other than the C-terminal regions, e.g., recognizing the N-terminal region, is preferably used in the primary reaction.

The monoclonal antibody of the present invention may be used in an assay system other than the sandwich method, such as a competitive method, an immunometric method and a nephrometry.

In the competitive method, an antigen in a test solution and a labeled antigen are competitively reacted with an antibody, then an unreacted labeled antigen (F) and a labeled antigen bound to the antibody (B) are separated (B/F separation) and the labeled amount of either B or F is measured to determine the amount of the antigen in the test solution. In the reactions for such a method, there are a liquid phase method in which a soluble antibody is used as the antibody and the B/F separation is effected by polyethylene glycol while a second antibody to the antibody is used, and a solid phase method in which an immobilized antibody is used as the first antibody or a soluble antibody is used as the first antibody while an immobilized antibody is used as the second antibody.

In the immunometric method, an antigen in a test solution and an immobilized antigen are competitively reacted with a given amount of a labeled antibody followed by separating the solid phase from the liquid phase; or an antigen in a test solution and an excess amount of labeled antibody are reacted, then an immobilized antigen is added to bind an unreacted labeled antibody to the solid phase and the solid phase is separated from the liquid phase. Thereafter, the labeled amount of any of the phases is measured to determine the antigen amount in the test solution.

In the nephrometry, the amount of insoluble sediment, which is produced as a result of the antigen-antibody reaction in a gel or in a solution, is measured. Even when the amount of an antigen in a test solution is small and only a small amount of the sediment is obtained, a laser nephrometry utilizing laser scattering can be suitably used.

In applying each of those immunoassays to the assay method of the present invention, any special conditions or operations are not required to set forth. The assay system for the protein of the present invention or salts thereof may be constructed in addition to conditions or operations conventionally used for each of the methods, taking the technical consideration of one skilled in the art into account. For the details of such conventional technical means, a variety of reviews, reference books, etc. may be referred.

See, for example, Hiroshi Irie (ed.): "Radioimmunoassay" (published by Kodansha, 1974); Hiroshi Irie (ed.): "Radioimmunoassay; Second Series" (published by Kodansha, 1979); Eiji Ishikawa, et al. (ed.): "Enzyme Immunoassay" (published by Igaku Shoin, 1978); Eiji Ishikawa, et al. (ed.): "Enzyme Immunoassay" (Second Edition) (published by Igaku Shoin, 1982); Eiji Ishikawa, et al. (ed.): "Enzyme Immunoassay" (Third Edition) (published by Igaku Shoin, 1987); "Methods in Enzymology" Vol. 70 (Immuochemical Techniques (Part A)); ibid., Vol. 73 (Immunochemical Techniques (Part B)); ibid., Vol. 74 (Immunochemical Techniques (Part C)); ibid., Vol. 84 (Immunochemical Techniques (Part D: Selected Immunoassays)); ibid., Vol. 92 (Immunochemical Techniques (Part E: Monoclonal Antibodies and General Immunoassay Methods)); ibid., Vol. 121 (Immunochemical Techniques (Part I: Hybridoma Technology and Monoclonal Antibodies)) (published by Academic Press); etc.]

As described above, the protein of the present invention or salts thereof can be quantified with high sensitivity, using the antibody of the present invention.

Furthermore, concentration of the protein of the present invention can be quantified, using the antibody of the present invention, thereby (1) when increase in concentration of the protein of the present invention is detected in a subject, diagnosis can be made as, for example, that a disease such as hyperglycemia or diabetes has occurred or the disease will highly probably affect the subject in the future and (2) when decrease in concentration of the protein of the present invention is detected, diagnosis can be made as, for example, that hypoglycemia has occurred or the disease will highly probably affect the subject in the future.

The antibody of the present invention can be employed for specifically detecting the protein of the present invention, which may be present in a test sample such as a body fluid, a tissue, etc. The antibody can also be used for preparation of an antibody column for purification of the protein of the present invention, detection of the protein of the present invention in the fractions upon purification, and analysis of the behavior of the protein of the present invention in the cells under investigation.

### (5) Gene diagnosis agent

The DNA of the present invention used as the probe, for example, allows detecting abnormal DNA or mRNA (gene abnormality) encoding the protein of the present invention in human or any other warm-blooded animals (e.g., rat, mouse, guinea pig, rabbit, fowl, sheep, swine, bovine, horse, cat, dog, monkey, chimpanzee, etc.) and. thus, useful as a gene diagnosis agent for diagnosing damage of the DNA or mRNA, mutation or expression or deterioration of expression, and increase or excessive expression of the DNA or mRNA.

Gene diagnosis as described above using the DNA of the present invention can be conducted by applying, for example, publicly known northern hybridization or PCR-SSCP method (Genomics, Vol. 5: 874-879 (1989); Proceedings of the National Academy of Sciences of the United States of America, 86, 2766-2770 (1989)), etc.)

For example, when excessive expression is detected by northern hybridization and when mutation of the DNA is detected by PCR-SSCP method, diagnosis can be highly probably made as, for example, that a disease such as hyperglycemia or diabetes, or hypoglycemia has occurred.

### (6) Pharmaceuticals containing antisense DNA.

An antisense DNA capable of binding complementarily to the DNA of the present invention and of suppressing expression of the DNA can suppress production of the protein of the present invention in a living body and hence, can be used as, for example, the prophylactic and therapeutic agent for hyperglycemia or diabetes, in the same way as that of the compound suppressing expression of the protein of the present invention as described above.

In the case where the aforementioned antisense DNA is used as the prophylactic and therapeutic agent as described above, operation can be performed in the same way as that of various prophylactic and therapeutic agents containing the aforementioned DNA of the present invention.

For example, in the case where the antisense DNA is used, operation can be conducted by using the antisense DNA independently or ordinary means after inserted into a proper vector such as retrovirus vector, adenovirus vector, adenovirus-associated virus vector, etc. The antisense DNA can be prepared in a drug form intactly or together with a physiologically acceptable carrier such as an auxiliary material for intake enhancement and, then, can be administered using a gene gun or such a catheter as a hydrogel catheter. Alternatively, it can be prepared in the form of an aerosol to administer as an inhalant into a trachea.

Further, the antisense DNA can be used as a diagnostic oligonucleotide probe to examine the presence in a tissue or a cell and a status of expression of the DNA of the present invention.

### (7) Pharmaceuticals containing the antibody of the present invention.

The antibody of the present invention having an action to neutralize an activity of the protein of the present invention can be used as pharmaceuticals (the prophylactic and therapeutic agents) for such diseases as hyperglycemia, diabetes, etc.

The prophylactic and therapeutic agents containing the antibody of the present invention for the aforementioned diseases can be orally or parenterally administered to a human and other warm-blooded animals (e.g., rat, rabbit, sheep, swine, bovine, cat, dog, monkey, etc.) as an intact solution or a pharmaceutical in a proper preparation form. A dose varies depending on a subject to be administered, a target disease, a symptom, a route for administration, etc.; when used for the purpose of prevention or treatment for, e.g., diabetes of adult, it is preferable that the antibody of the invention is intravenously administered normally in a daily dose of about 0.1 mg to about 20 mg/ kg body weight, preferably about 0.1 to about 10 mg/kg body weight, and more preferably about 0.1 to about 5 mg/kg body weight, once to 5 times a day and preferably once to 3 times a day. The dose near this case can be administered in case of other parenteral administration and oral administration. When a symptom is very severe, the dose may be increased in accordance with the symptom.

The antibody of the present invention can be administered intactly or as a proper pharmaceutical composition. The pharmaceutical composition used for the above-described administration contains the antibody of the present invention, the physiologically acceptable carrier, a diluting agent, or a vehicle. Such composition is provided in the orally or parentally suitable drug form.

That is, for example, the composition for oral administration is exemplified by a solid or liquid drug form, specifically a tablet (including sugar-coated and film-coated tables,) pill, granule, powder, capsule (including soft capsule,) syrup, emulsion, suspension, etc. Such composition is manufactured by a publicly known method and contains the carrier, diluting agent, or vehicle generally used in pharmaceutical field. For example, as the carrier and vehicle for the tablet, lactose, starch, sucrose, magnesium stearate, etc. are used.

The composition used for parenteral administration is, e.g., injection, suppositorium, etc. and injection includes forms such as intravenous injection, subcutaneous injection, endodermic injection, muscular injection, drop injection, etc. Such injection is prepared by dissolving, suspending, or emulsifying the antibody or its salt, for example, as described above in sterile aqueous or oleaginous solution ordinarily used for injection following the publicly known method. The aqueous solution used for injection are, for example, physiological saline, an isotonic solution containing glucose or any other auxiliary agents and may be used in combination with a proper dissolving aid such as alcohol (e.g., ethanol,) polyalcohol (e.g., propylene glycol, polyethylene glycol,) nonionic surfactant (e.g., polysorbate80, HCO-50 (polyoxyethylene (50 mol) adduct of hydrogenated castor oil).) The oleaginous solution used is, for example, sesame oil, soybean oil, etc. and benzyl benzoate, benzyl alcohol, etc. may be used in combination as the dissolving aid. As a rule, the injection prepared is filled in a proper ampoule. The suppositorium used for rectum administration is prepared by compounding the antibody or its salt as described above with a normal base material for the suppositorium.

It is advantageous to prepare the above-described pharmaceutical compound for an oral or parenteral use in the drug form in an administration unit compatible with the administrating amount of an active component. The drug forms of such administration unit are exemplified by the table, pill, capsule, injection (ampoule,) suppositorium, etc. It is preferable that normally 5-500 mg is contained in each administration unit drug form, particularly 5-10 mg in the injection, 10-250 mg in other drug forms.

Individual composition as described before may contain other active components unless any undesirable interactions are caused by compounding with the aforementioned antibody.

### (8) DNA transferred Animals

The present invention provides a non-human mammal having DNA (hereafter exogenous mutant DNA of the present invention) encoding an exogenous protein of the present invention or its mutant DNA (hereafter exogenous DNA of the present invention.)

That is, the present invention provides
(1) a non-human mammal having an exogenous DNA of the present invention or its mutant DNA,
(2) the animal of claim 1, wherein the non-human mammal is a rodent,
(3) the animal of claim 1, wherein the rodent is a mouse or a rat, and
(4) a recombinant vector containing an exogenous DNA of the present invention or its mutant DNA and expressible in a mammal.

The non-human mammal having the exogenous DNA of the present invention or its mutant DNA (hereafter a DNA transfer animal of the present invention) can be produced by transferring an objective DNA preferably to an unfertilized egg, fertilized egg, sperm, and a germinative cell including its initial cell in a stage of ontogenesis '(more preferably, the unicellular amphicytula stage generally before an 8-cell stage) in ontogenesis of the non-human mammal by calcium phosphate method, electric pulse method, lipofection method, microinjection method, particle gun method, DEAE dextran method, retrovirus method, etc. On the other hand, the DNA transfer method enables a use of the objective exogenous DNA of the present invention for a cell culture and tissue culture by transferring it to a somatic cell, an organ of a living body, a tissue cell. In addition, the DNA transferred animal of the present invention can be prepared by fusing these cells with each other applying the aforementioned germinative cell and a publicly known cell fusion method.

Non-human mammals used are exemplified by rats, mice, rabbits, sheep, swine, bovine, cats, dogs, goats, Guinea pigs, hamsters, or the like. Particularly, in consideration of preparation of an ill health animal model, rodents, which are relatively short in ontogenesis and a biological cycle and easy to breed, particularly mouse (e.g., pure lies of C57, BL/6 line, DBA2 line, etc.; hybrid lines such as B6C3F1 line, BDF1 line, B6D2F1 line BALB/c line, ICR line, etc.) or rats (e.g., Wistar, SD, etc.) are preferable.

"Mammals" in a recombinant vector that can be expressed in the mammals include the aforesaid non-human mammals and human.

The exogenous DNA of the invention refers to the DNA of the invention that is once isolated and extracted from mammals, not the DNA of the invention inherently possessed by the non-human mammals.

The mutant DNA of the invention includes mutants resulting from variation (e.g., mutation, etc.) in the base sequence of the original DNA of the invention, specifically DNAs resulting from base addition, deletion, substitution with other bases, etc. and further including abnormal DNA.

The abnormal DNA is intended to mean DNA that expresses the abnormal protein of the invention and exemplified by the DNA that expresses a protein for suppressing the function of the normal protein of the invention.

The exogenous DNA of the invention may be any one of those derived from a mammal of the same species as, or a different species from, the mammal as the target animal. To transfer the DNA of the present invention to a target animal, it is generally advantageous to use the DNA in a DNA construct ligated downstream a promoter capable of expressing the DNA in an animal cell. For example, when a human DNA of the present invention is transferred, the DNA construct (e.g., vector etc.,) in which the DNA of the present invention is ligated downstream various promoters that can expresses the mammal (rabbits, dogs, cats, Guinea pigs, hamsters, rats, mice, or the like) -derived DNA having the DNA of the present invention highly homologous thereto, is microinjected to the fertilized egg of the target mammal, e.g., a fertilized fertilized egg of a mouse. Thus, the DNA transfer mammal capable of producing a high level of the receptor protein or the like of the present invention can be prepared.

Examples of the expression vector of the present invention which can be used include plasmids derived form E. coli, plasmids derived from Bacillus subtilis, plasmids derived from yeast, bacteriophages such as λ phage, etc., animal viruses such as retrovirus exemplified by Moloney leukemia virus, vaccinia virus, baculovirus, etc. Among these vectors, plasmids derived form E. coli, plasmids derived from Bacillus subtilis, plasmids derived from yeast, etc. are preferably used.

Examples of these promoters for regulating the DNA expression include (1) promoters for DNA derived from viruses (e.g., simian virus, cytomegalovirus, Moloney leukemia virus, JC virus, breast cancer virus, poliovirus, etc.), and (2) promoters derived from various mammals (human, rabbits, dogs, cats, guinea pigs, hamsters, rats, mice, etc.), for example, promoters of albumin, insulin II, uroplakin II, elastase, erythropoietin, endothelin, muscular creatine kinase, glial fibrillary acidic protein, glutathione S-transferase, platelet-derived growth factor β, keratins K1, K10 and K14, collagen types I and II, cyclic AMP-dependent protein kinase βI subunit, dystrophin, tartarate-resistant alkaline phosphatase, atrial natriuretic factor, endothelial receptor tyrosine kinase (generally abbreviated as Tie2), sodium-potassium adenosine triphosphorylase (Na,K-ATPase), neurofilament light chain, metallothioneins I and IIA, metalloproteinase I tissue inhibitor, MHC class I antigen (H-2L), H-ras, renin, dopamine β-hydroxylase, thyroid peroxidase (TPO), protein chain elongation factor 1α (EF-1α), β actin, α and β myosin heavy chains, myosin light chains 1 and 2, myelin base protein, thyroglobulins, Thy-1, immunoglobulins, H-chain variable region (VNP), serum amyloid component P, myoglobin, troponin C, smooth muscle α actin, preproencephalin A, vasopressin, etc. Among them, cytomegalovirus promoters, human protein elongation factor 1α (EF-1α) promoters, human and chicken β actin promoters, etc., which are capable of high expression in the whole body are preferred.

Preferably, the vectors described above have a sequence that terminates the transcription of the desired messenger RNA in the DNA transgenic animal (generally termed terminator); for example, a sequence of each DNA derived from viruses and various mammals, and SV40 terminator of the simian virus and the like are preferably used.

In addition, for the purpose of increasing the expression of the desired exogenous DNA to a higher level, the splicing signal and enhancer region of each DNA, a portion of the intron of an eukaryotic DNA may also be ligated at the 5' upstream of the promoter region, or between the promoter region and the translational region, or at the 3' downstream of the translational region, depending upon purposes.

The translational region for the normal protein of the invention can be obtained using as a starting material the entire genomic DNA or its portion of liver, kidney, thyroid cell or fibroblast origin from human or various mammals (e.g., rabbits, dogs, cats, guinea pigs, hamsters, rats, mice, etc.) or of various commercially available genomic DNA libraries, or using cDNA prepared by a publicly known method from RNA of liver, kidney, thyroid cell or fibroblast origin as a starting material. Also, an exogenous abnormal DNA can produce the translational region through variation of the translational region of normal protein obtained from the cells or tissues described above by point mutagenesis.

The translational region can be prepared by a conventional genetic engineering technique in which the DNA is ligated downstream the aforesaid promoter and if desired, upstream the translation termination site, as a DNA construct capable of being expressed in the transgenic animal.

The exogenous DNA of the invention is transfected at the amphicytula stage in a manner such that the DNA is certainly present in all the germinal cells and somatic cells of the target mammal. The fact that the exogenous DNA of the invention is present in the germinal cells of the animal prepared by DNA transfection means that all offspring of the prepared animal will maintain the exogenous DNA of the invention in all of the germinal cells and somatic cells thereof. The offspring of the animal that inherits the exogenous DNA of the invention also have the exogenous DNA in all of the germinal cells and somatic cells thereof.

The non-human mammal in which the normal exogenous DNA of the invention has been transfected can be passaged as the DNA-bearing animal under ordinary rearing environment, by confirming that the exogenous DNA is stably retained by mating.

By the transfection of the exogenous DNA of the invention at the amphicytula stage, the DNA is retained to be excess in all of the germinal and somatic cells. The fact that the exogenous DNA of the invention is excessively present in the germinal cells of the prepared animal after transfection means that the DNA of the invention is excessively present in all of the germinal cells and somatic cells thereof. The offspring of the animal that inherits the exogenous DNA of the invention have excessively the DNA of the invention in all of the germinal cells and somatic cells thereof.

By obtaining a homozygous animal having the transfected DNA in both of homologous chromosomes and mating a male and female of the animal, all offspring can be passaged to retain the DNA.

In a non-human mammal bearing the normal DNA of the invention, the normal DNA of the invention has expressed to a high level, and may eventually develop the hyperfunction of the protein of the invention by promoting the functions of endogenous normal DNA. Therefore, the animal can be utilized as a pathologic model animal for such a disease. Specifically, using the normal DNA transgenic animal of the invention, it is possible to elucidate the mechanism of the hyperfunction of the protein of the invention and the pathological mechanism of the disease associated with the protein of the invention and to determine how to treat the disease.

Furthermore, since a mammal transfected with the exogenous normal DNA of the invention exhibits an increasing symptom of the protein of the invention librated, the animal is usable for screening of therapeutic agents for the disease associated with the protein of the invention.

On the other hand, non-human mammal having the exogenous abnormal DNA of the invention can be passaged under normal breeding conditions as the exogenous abnormal DNA-bearing animal by confirming the stable retention of the exogenous DNA via crossing. Moreover, the objective exogenous DNA can be utilized as a starting material by inserting the DNA into the plasmid described above. The DNA construct with a promoter can be prepared by conventional DNA engineering techniques. The transfection of the abnormal DNA of the invention at the amphicytula stage is preserved to be present in all of the germinal and somatic cells of the target mammal. The fact that the abnormal DNA of the invention is present in the germinal cells of the animal after DNA transfection means that all of the offspring of the animal prepared have the abnormal DNA of the invention in all of the germinal and somatic cells. ' Such an offspring which passaged the exogenous DNA of the invention retains the abnormal DNA of the invention in all of the germinal and somatic cells. By obtaining a homozygous animal having the transfected DNA in both of homologous chromosomes and mating a male and female of the animal, all offspring can be passaged to retain the DNA.

Since non-human mammal having the abnormal DNA of the invention may express the abnormal DNA of the invention at a high level, the animal may sometimes be the function inactivation type inadaptability to the protein of the invention by inhibiting the function of the endogenous normal DNA and can be utilized as its disease model animal. For example, using the abnormal DNA transgenic animal of the invention, it is possible to elucidate the mechanism of inadaptability to the protein of the invention and to perform to study a method for treatment of this disease.

More specifically, the animal of the invention expressing the abnormal DNA of the invention to a high level is also expected to serve as an experimental model to elucidate the mechanism of the functional inhibition (dominant negative effect) of normal protein by the abnormal protein of the invention in the function inactive type inadaptability to the protein of the invention.

A mammal received the abnormal exogenous DNA of the invention is also expected to serve for screening a candidate drug for the treatment of the function inactive type inadaptability to the protein of the invention, since the protein of the invention increases in such an animal in its free form.

Other potential applications of two kinds of the transgenic animals described above include:
(1) use as a cell source for tissue culture;
(2) elucidation of the relation to a protein that is specifically expressed or activated by the protein of the invention, by direct analysis of DNA or RNA in tissues of the DNA transgenic animal of the invention or by analysis of the protein tissues expressed by the DNA;
(3) research in the function of cells derived from tissues that are usually cultured only with difficulty, using cells in tissues bearing the DNA cultured by a standard tissue culture technique;
(4) screening a drug that enhances the functions of cells using the cells described in (3) above; and,
(5) isolation and purification of the variant protein of the invention and preparation of an antibody thereto.

Furthermore, clinical conditions of a disease associated wit the protein of the invention, including the function inactive type inadaptability to the protein of the protein of the invention can be determined by using the DNA transgenic animal of the invention. Also, pathological findings on each organ in a disease model associated with the protein of the invention can be obtained in more detail, leading to the development of a new method for treatment as well as the research and therapy of any secondary diseases associated with the disease.

It is also possible to obtain a free cell, in which the DNA is transfected, by withdrawing each organ from the DNA transgenic animal of the invention, mincing the organ and degrading with a proteinase such as trypsin, etc., followed by establishing the line of culturing or cultured cells. Furthermore, the DNA transgenic animal of the invention can serve to identify cells capable of producing the protein of the invention, and to study in association with apoptosis, differentiation or propagation or on the mechanism of signal transduction in these properties to inspect any abnormality therein. Thus, the DNA transgenic animal of the invention can provide an effective research material for the protein of the invention and for elucidation of the function and effect thereof.

To develop a drug for the treatment of diseases associated with the protein of the invention, including the function inactive type inadaptability to the protein of the invention, using the DNA transgenic animal of the invention, an effective and rapid method for screening can be provided by using the method for inspection and the method for quantification, etc. described above. It is also possible to investigate and develop a method for DNA therapy for the treatment of diseases associated with the protein of the invention, using the DNA transgenic animal of the invention or a vector capable of expressing the exogenous DNA of the invention.

### (9) Knockout animal

The present invention provides a non-human mammal embryonic stem cell bearing the DNA of the invention inactivated and a non-human mammal deficient in expressing the DNA of the invention.

Thus, the present invention provides:
(1) a non-human embryonic stem cell in which the DNA of the invention is inactivated;
(2) the embryonic stem cell according to (1), wherein the DNA is inactivated by introducing a drug resistance gene (e. g., neomycin resistance gene) or a reporter gene (e.g., β-galactosidase gene derived from Escherichia coli);
(3) the embryonic stem cell according to (1), which is resistant to neomycin;
(4) the embryonic stem cell according to (1), wherein the non-human mammal is a rodent;
(5) the embryonic stem cell according to (4), wherein the rodent is mouse;
(6) a non-human mammal deficient in expressing the DNA of the invention, wherein the DNA of the invention is inactivated;
(7) the non-human mammal according to (5), wherein the DNA is inactivated by inserting the drug resistance gene (e. g., neomycin resistance gene) or the reporter gene (e.g., β-galactosidase derived from Escherichia coli) therein and the reporter gene is capable of being expressed under control of a promoter for the DNA of the invention;
(8) the non-human mammal according to (6), which is a rodent;
(9) the non-human mammal according to (8), wherein the rodent is mouse; and,
(10) a method of screening a compound that promotes or inhibits the promoter activity for the DNA of the invention, which comprises administering a test compound to the mammal of (7) and detecting expression of the drug resistance gene or the reporter gene.

The non-human mammal embryonic stem cell in which the DNA of the invention is inactivated refers to a non-human mammal embryonic stem cell that suppresses the ability of the non-human mammal to express the DNA by artificially mutating the DNA, or the DNA has no substantial ability to express the protein of the invention (hereafter sometimes referred to as the knockout DNA of the invention) by substantially inactivating the activities of the protein of the invention encoded by the DNA (hereafter merely referred to as ES cell).

The non-human mammals used are similar to those as described above.

Techniques for artificially mutating the DNA of the invention include deletion of a part or all of the DNA sequence and insertion of or substitution with other DNA, by genetic engineering. By these variations, the knockout DNA of the invention may be prepared, for example, by shifting the reading frame of a codon or by disrupting the function of a promoter or exon.

Specifically, the non-human mammal embryonic stem cell in which the DNA of the invention is inactivated (hereafter merely referred to as the ES cell with the DNA of the invention inactivated or the knockout ES cell of the invention) can be obtained by, for example, isolating the DNA of the invention that the desired non-human mammal possesses, inserting a DNA fragment having a DNA sequence constructed by inserting a drug resistant gene such as a neomycin resistant gene or a hygromycin resistant gene, or a reporter gene such as lacZ (β-galactosidase gene) or cat (chloramphenicol acetyltransferase gene), etc. into its exon site thereby to disable the functions of exon, or integrating to a chromosome of the target animal by, e.g., homologous recombination; a DNA sequence that terminates gene transcription (e.g., polyA additional signal, etc.) in the intron between exons, thus inhibiting the synthesis of complete messenger RNA and eventually destroying the gene (hereafter simply referred to as targeting vector). The thus-obtained ES cells to the Southern hybridization analysis with a DNA sequence on or near the DNA of the invention as a probe, or to PCR analysis with a DNA sequence on the targeting vector and another DNA sequence near the DNA of the invention which is not included in the targeting vector as primers, to select the knockout ES cell of the invention.

The parent ES cells to inactivate the DNA of the invention by homologous recombination, etc. may be of a strain already established as described above, or may be originally established in accordance with a modification of the publicly known method by Evans and Kaufman supra. For example, in the case of mouse ES cells, currently it is common practice to use ES cells of the 129 strain. However, since their immunological background is obscure, the C57BL/6 mouse or the BDF1 mouse (F1 hybrid between C57BL/6 and DBA/2), wherein the low ovum availability per C57BL/6 in the C57BL/6 mouse has been improved by crossing with DBA/2, may be preferably used, instead of obtaining a pure line of ES cells with the clear immunological genetic background and for other purposes. The BDF1 mouse is advantageous in that, when a pathologic model mouse is generated using ES cells obtained therefrom, the genetic background can be changed to that of the C57BL/6 mouse by back-crossing with the C57BL/6 mouse, since its background is of the C57BL/6 mouse, as well as being advantageous in that ovum availability per animal is high and ova are robust.

In establishing ES cells, blastocytes at 3.5 days after fertilization are commonly used. In the present invention, embryos are preferably collected at the 8-cell stage, after culturing until the blastocyte stage, the embryos are used to efficiently obtain a large number of early stage embryos.

Although the ES cells used may be of either sex, male ES cells are generally more convenient for generation of a germ cell line chimera and are therefore preferred. It is also desirable that sexes are identified as soon as possible to save painstaking culture time.

Methods for sex identification of the ES cell include the method in which a gene in the sex-determining region on the Y-chromosome is amplified by the PCR process and detected. When this method is used, one colony of ES cells (about 50 cells) is sufficient for sex-determination analysis, which karyotype analysis, for example G-banding method, requires about 10⁶ cells; therefore, the first selection of ES cells at the early stage of culture can be based on sex identification, and male cells can be selected early, which saves a significant amount of time at the early stage of culture.

Second selection can be achieved by, for example, confirmation of the number of chromosomes by the G-banding method. It is usually desirable that the chromosome number of the obtained ES cells be 100% of the normal number. However, when it is difficult to obtain the cells having the normal number of chromosomes due to physical operations, etc. in the cell establishment, it is desirable that the ES cell is again cloned to a normal cell (e.g., in a mouse cell having the number of chromosomes being 2n = 40) after knockout of the gene of the ES cells.

Although the embryonic stem cell line thus obtained shows a very high growth potential, it must be subcultured with great care, since it tends to lose its ontogenic capability. For example, the embryonic stem cell line is cultured at about 37°C in a carbon dioxide incubator (preferably about 5% carbon dioxide and about 95% air, or about 5% oxygen, about 5% carbon dioxide and 90% air) in the presence of LIF (1 to 10000 U/ml) on appropriate feeder cells such as STO fibroblasts, treated with a trypsin/EDTA solution (normally about 0.001 to about 0.5% trypsin/about 0.1 to about 5 mM EDTA, preferably about 0.1% trypsin/1 mM EDTA) at the time of passage to obtain separate single cells, which are then seeded on freshly prepared feeder cells. This passage is normally conducted every 1 to 3 days; it is desirable that cells be observed at passage and cells found to be morphologically abnormal in culture, if any, be abandoned.

Where ES cells are allowed to reach a high density in mono-layers or to form cell aggregates in suspension under appropriate conditions, they will spontaneously differentiate to various cell types, for example, pariental and visceral muscles, cardiac muscle or the like [M. J. Evans and M. H. Kaufman, Nature, 292, 154, 1981; G. R. Martin, Proc. Natl. Acad. Sci. U.S.A., 78, 7634, 1981; T. C. Doetschman et al., Journal of Embryology Experimental Morphology, 87, 27, 1985]. The cells deficient in expression of the DNA of the invention, which are obtained from the differentiated ES cells of the invention, are useful for studying the function of the protein of the invention cytologically or molecular biologically.

The non-human mammal deficient in expression of the DNA of the invention can be identified from a normal animal by measuring the mRNA amount in the subject animal by a publicly known method, and indirectly comparing the degrees of expression.

The non-human mammals used are similar to those as described above.

With respect to the non-human mammal deficient in expression of the DNA of the invention, the DNA of the invention can be made knockout by transfecting a targeting vector, prepared as described above, to non-human mammal embryonic stem cells or oocytes thereof, and conducting homologous recombination in which a targeting vector DNA sequence, wherein the DNA of the invention is inactivated by the transfection, is replaced with the DNA of the invention on a chromosome of a non-human mammal embryonic stem cell or embryo thereof.

The knockout cells with the disrupted DNA of the invention can be identified by the Southern hybridization analysis using as a probe a DNA fragment on or near the DNA of the invention, or by the PCR analysis using as primers a DNA sequence on the targeting vector and another DNA sequence which is not included in the targeting vector. When non-human mammalian embryonic stem cells are used, a cell line wherein the DNA of the invention is inactivated by homologous recombination is cloned; the resulting clones are injected to, e.g., a non-human mammalian embryo or blastocyst, at an appropriate stage such as the 8-cell stage. The resulting chimeric embryos are transplanted to the uterus of the pseudopregnant non-human mammal. The resulting animal is a chimeric animal constructed with both cells having the normal locus of the DNA of the invention and those having an artificially mutated locus of the DNA of the invention.

When some germ cells of the chimeric animal have a mutated locus of the DNA of the invention, an individual, which entire tissue is composed of cells having a mutated locus of the DNA of the invention can be selected from a series of offspring obtained by crossing between such a chimeric animal and a normal animal, e.g., by coat color identification, etc. The individuals thus obtained are normally deficient in heterozygous expression of the peptide of the invention. The individuals deficient in homozygous expression of the protein of the invention can be obtained from offspring of the intercross between the heterozygotes.

When an oocyte or egg cell is used, a DNA solution may be injected, e.g., to the prenucleus by microinjection thereby to obtain a transgenic non-human mammal having a targeting vector introduced in a chromosome thereof. From such transgenic non-human mammals, those having a mutation at the locus of the DNA of the invention can be obtained by selection based on homologous recombination.

As described above, individuals in which the DNA of the invention is rendered knockout permit passage rearing under ordinary rearing conditions, after the individuals obtained by their crossing have proven to have been knockout.

Furthermore, the genital system may be obtained and maintained by conventional methods. That is, by crossing male and female animals each having the inactivated DNA, homozygote animals having the inactivated DNA in both loci can be obtained. The homozygotes thus obtained may be reared so that one normal animal and two or more homozygotes are produced from a mother animal to efficiently obtain such homozygotes. By crossing male and female heterozygotes, homozygotes and heterozygotes having the inactivated DNA are proliferated and passaged.

The non-human mammal embryonic stem cell, in which the DNA of the invention is inactivated, is very useful for preparing a non-human mammal deficient in expression of the DNA of the invention.

Since the non-human mammal, in which the DNA of the invention is inactivated, lacks various biological activities derived from the protein of the invention, such an animal can be a disease model suspected of inactivated biological activities of the protein of the invention and thus, offers an effective study to investigate the causes for and therapy for these diseases. (10) Method of screening a compound having a therapeutic/prophylactic effect on diseases caused by deficiency, damages, etc. of the DNA of the invention

The non-human mammal deficient in expression of the DNA of the invention can be employed for screening of a compound having a therapeutic/prophylactic effect on diseases (e.g., hypoglycemia, etc.) caused by deficiency, damages, etc. of the DNA of the invention.

That is, the present invention provides a method of screening a compound having a therapeutic/prophylactic effect on diseases caused by deficiency, damages, etc. of the DNA of the invention, which comprises administering a test compound to the non-human mammal deficient in expression of the DNA of the invention and observing and measuring a change occurred in the animal.

As the non-human mammal deficient in expression of the DNA of the invention that can be employed for the screening method, the same examples as given hereinabove apply.

Examples of the test compound include peptides, proteins, non-peptide compounds, synthetic compounds, fermentation products, cell extracts, plant extracts, animal tissue extracts, blood plasma and the like and these compounds may be novel compounds or publicly known compounds.

Specifically, the non-human mammal deficient in expression of the DNA of the invention is treated with a test compound, comparison is made with an intact animal for control and a change in each organ, tissue, disease conditions, etc. of the animal is used as an index to assess the therapeutic/prophylactic effects of the test compound.

For treating an animal to be tested with a test compound, for example, oral administration, intravenous injection, etc. are applied and the treatment can be appropriately selected depending upon conditions of the test animal, properties of the test compound, etc. Furthermore, a dose of the test compound to be administered can be selected depending on the administration route, nature of the test compound, etc.

The compound obtained using the above screening method is a compound selected from the test compounds described above and exhibits a therapeutic and prophylactic effect on a disease (e.g., hypoglycemia, etc.) caused by low expression of the protein of the invention. Therefore, the compound can be employed as a safe and low toxic drug for the treatment and prevention of the disease. Furthermore, compounds derived from the compound obtained by the screening supra can be likewise employed.

The compound obtained by the screening method above may form salts, and may be used in the form of salts with physiologically acceptable acids (e.g., inorganic acids or organic acids) or bases (e.g., alkali metal salts), preferably in the form of physiologically acceptable acid addition salts. Examples of such salts are salts with inorganic acids (e.g., hydrochloric acid, phosphoric acid, hydrobromic acid, sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

A pharmaceutical comprising the compound obtained by the above screening method or salts thereof may be manufactured in a manner similar to the method for preparing the pharmaceutical comprising the protein of the invention described hereinabove. Since the pharmaceutical obtained is safe and low toxic, it can be administered to human or any other warm-blooded animals (e.g., rat, mouse, guinea pig, rabbit, sheep, swine, bovine, horse, cat, dog, monkey, etc.)

A dose of the compound or its salt to be administered varies depending upon target disease, subject to be administered, route of administration, etc. in general; when the compound is orally administered for the purpose of treatment for, e.g., arteriosclerosis, the compound is administered to adult (as 60 kg body weight) in a daily dose of about 0.1 to 100 mg, preferably about 1.0 to 50 mg, more preferably about 1.0 to 20 mg. For parenteral administration to an adult (as 60 kg body weight), a single dose may vary depending upon subject to be administered, target disease, etc., but when the compound is administered in the form of an injectable preparation for the purpose of treatment for, e.g., arteriosclerosis, it is advantageous to administer the compound intravenously to an adult (as 60 kg body weight) in a daily dose of about 0.01 to 30 mg, preferably about 0.1 to 20 mg, more preferably about 0.1 to 10 mg, though the single dosage varies depending upon particular subject, particular disease, etc. As for other animals, the composition can be administered in the above amount with converting it into that for the body weight of 60 kg.

### (11) Method of screening a compound that promotes or inhibits the activity of a promoter to the DNA of the invention

The present invention provides a method of screening a compound or its salts that promote or inhibit the activity of a promoter to the DNA of the invention, which comprises administering a test compound to a non-human mammal deficient in expression of the DNA of the invention and detecting the expression of the reporter gene.

In the screening method above, the non-human mammal deficient in expression of the DNA of the invention is selected from the aforesaid non-human mammal deficient in expression of the DNA of the invention, as an animal in which the DNA of the invention is inactivated by introducing a reporter gene and the reporter gene is expressed under control of a promoter to the DNA of the invention.

The same examples of the test compound apply to specific compounds used for the screening.

As the reporter gene, the same specific examples apply to this screening method. Preferably employed are β-galactosidase (lacZ), soluble alkaline phosphatase gene, luciferase gene and the like. .

Since a reporter gene is present under control of a promoter to the DNA of the invention in the non-human mammal deficient in expression of the DNA of the invention wherein the DNA of the invention is substituted with the reporter gene, the activity of the promoter can be detected by tracing expression of a substance encoded by the reporter gene.

When a part of the DNA region encoding the protein of the invention is substituted with, e.g., β-galactosidase gene (lacZ) derived from Escherichia coli, β-galactosidase is expressed in a tissue where the protein of the invention should originally be expressed, instead of the protein of the invention. Thus, the state of expression of the protein of the invention can be readily observed in vivo of an animal by staining with a reagent, e.g., 5-bromo-4-chloro-3-indolyl-β-galactopyranoside (X-gal) that is substrate for β-galactosidase. Specifically, a mouse deficient in the protein of the invention, or its tissue section is fixed with glutaraldehyde, etc. After washing with phosphate buffered saline (PBS), the system is reacted with a staining solution containing X-gal at room temperature or about 37°C for approximately 30 minutes to an hour. After the β-galactosidase reaction is terminated by washing the tissue preparation with 1 mM EDTA/PBS solution, the color formed is observed. Alternatively, mRNA encoding lacZ may be detected in a conventional manner.

The compounds or salts thereof obtained using the screening method as described above are compounds that are selected from the test compounds described above and that promote or inhibit the promoter activity to the DNA of the invention.

The compound obtained by the screening method above may form salts, and may be used in the form of salts with physiologically acceptable acids (e.g., inorganic acids or organic acids) or bases (e.g., alkali metal salts), preferably in the form of physiologically acceptable acid addition salts. Examples of such salts are salts with inorganic acids (e.g., hydrochloric acid, phosphoric acid, hydrobromic acid, sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

Since the compound or its salts that promote the promoter activity to the DNA of the present invention can promote the expression of the protein of the invention and promote the activity of the protein, they are useful as safe and low toxic drugs for the treatment/prevention of diseases such as hypoglycemia, etc.

On the other hand, since the compound or its salts that inhibits the promoter activity to the DNA of the present invention can inhibit the expression of the protein of the invention and inhibit the activity of the protein, they are useful as safe and low toxic drugs for the treatment/prevention of diseases such as hyperglycemia, diabetes, etc.

A pharmaceutical comprising the compound obtained by the above screening method or salts thereof may be manufactured in a manner similar to the method for preparing the pharmaceutical comprising the protein of the invention described hereinabove. Since the pharmaceutical product thus obtained is safe and low toxic, it can be administered to, for example, human or any other warm-blooded animals (e.g., rat, mouse, guinea pig, rabbit, sheep, swine, bovine, horse, cat, dog, monkey, etc.).

A dose of the compound or its salt to be administered varies depending upon target disease, subject to be administered, route of administration, etc. in general; when the compound inhibiting the promoter activity to the DNA of the invention is orally administered for the purpose of treatment for, e.g., diabetes, the compound is administered to adult (as 60 kg body weight) in a daily dose of about 0.1 to 100 mg, preferably about 1.0 to 50 mg, more preferably about 1.0 to 20 mg. For parenteral administration to an adult (as 60 kg body weight), a single dose varies depending upon subject to be administered, target disease, etc., but when the compound is administered in the form of an injectable preparation for the purpose of treatment for, e.g., diabetes, it is advantageous to administer the compound inhibiting the promoter activity to the DNA of the invention intravenously to an adult (as 60 kg body weight) in a daily dose of about 0.01 to 30 mg, preferably about 0.1 to 20 mg, more preferably about 0.1 to 10 mg. As for other animals, the composition can be administered in the above amount with converting it into that for the body weight of 60 kg.

A dose of the compound or salts thereof varies depending on target disease, subject to be administered, route for administration, etc.; when the compound that promotes the promoter activity to the DNA of the invention is orally administered for the purpose of treatment for, e.g., hypoglycemia, the compound is administered to adult (as 60 kg body weight) normally in a daily dose of about 0.1 to 100 mg, preferably about 1.0 to 50 mg, more preferably about 1.0 to 20 mg. In parenteral administration, a single dose of the compound varies depending on subject to be administered, target disease, etc. but when the compound of promoting the promoter activity to the protein of the invention is administered to adult (as 60 kg body weight) in the form of injectable preparation for the purpose of treating hypoglycemia, it is advantageous to administer the compound intravenously in a daily dose of about 0.01 to about 30 mg, preferably about 0.1 to about 20 mg, more preferably about 0.1 to about 10 mg. For other animal species, the corresponding dose as converted per 60 kg weight can be administered.

As stated above, the non-human mammal deficient in expression of the DNA of the present invention is extremely useful for screening the compound or its salt that promotes or inhibits the promoter activity to the DNA of the invention and can greatly contribute to elucidation of causes for various diseases suspected of deficiency in expression of the DNA of the invention and for the development of prophylactic/therapeutic drug for these diseases.

Furthermore, a so-called transgenic animal (gene transferred animal) can be prepared by using DNA containing the promoter region of the protein of the invention, ligating genes encoding various proteins at the downstream and injecting the same into oocyte of an animal. It is then possible to synthesize the protein therein specifically and study its activity in vivo. When an appropriate reporter gene is ligated to the promoter site above and a cell line that expresses the gene is established, the resulting system can be utilized as the search system for a low molecular compound having the action of specifically promoting or inhibiting the in vivo productivity of the protein of the invention itself. Moreover, it is possible to find a novel cis-element and a transcription factor bound to the cis-element by analyzing the promoter portion.

In the specification and drawings, the codes of bases, amino acids, etc. are denoted in accordance with the IUPAC-IUB Commission on Biochemical Nomenclature or by the common codes in the art, examples of which are shown below. For amino acids that may have the optical isomer, L form is presented unless otherwise indicated.
- DNA: : deoxyribonucleic acid
- cDNA: : complementary deoxyribonucleic acid
- A: : adenine
- T: : thymine
- G: : guanine
- C: : cytosine
- RNA: : ribonucleic acid
- mRNA: : messenger ribonucleic acid
- dATP: : deoxyadenosine triphosphate
- dTTP: : deoxythymidine triphosphate
- dGTP: : deoxyguanosine triphosphate
- dCTP: : deoxycytidine triphosphate
- ATP: : adenosine triphosphate
- EDTA: : ethylenediaminetetraacetic acid
- SDS: : sodium dodecyl sulfate
- Gly: : glycine
- Ala: : alanine
- Val: valine
- Leu: : leucine
- Ile: : isoleucine
- Ser: : serine
- Thr: : threonine
- Cys: : cysteine
- Met: : methionine
- Glu: : glutamic acid
- Asp: : aspartic acid
- Lys: : lysine
- Arg: : arginine
- His: : histidine
- Phe: : phenylalanine
- Tyr: : tyrosine
- Trp: : tryptophan
- Pro: : proline
- Asn: : asparagine
- Gln: : glutamine
- pGlu: : pyroglutamic acid

Substituents, protecting groups and reagents generally used in this specification are presented as the codes below.
- Me: : methyl
- Et: : ethyl
- Bu: : butyl
- Ph: : phenyl
- TC: : thiazolidine-4(R)-carboxamido
- Tos: : p-toluenesulfonyl
- CHO: : formyl
- Bzl: : benzyl
- Cl₂-Bzl: : 2,6-dichlorobenzyl
- Bom: : benzyloxymethyl
- Z: : benzyloxycarbonyl
- Cl-Z: : 2-chlorobenzyloxycarbonyl
- Br-Z: : 2-bromobenzyl oxycarbonyl
- Boc: : t-butoxycarbonyl
- DNP: : dinitrophenol
- Trt: : trityl
- Bum: : t-butoxymethyl
- Fmoc: : N-9-fluorenyl methoxycarbonyl
- HOBt: : 1-hydroxybenztriazole
- HOOBt: : 3,4-dihydro-3-hydroxy-4-oxo-1,2,3-benzotriazine
- HONB: : 1-hydroxy-5-norbornene-2,3-dicarboxyimide
- DCC: : N,N'-dichlorohexylcarbodiimide

The sequence identification numbers in the sequence listing of the specification indicates the following sequence, respectively.

### [SEQ ID NO: 1]

This shows the amino acid sequence of human MD25 (VLCAD).

### [SEQ ID NO: 2]

This shows base sequence of human MD25 (VLCAD) gene (cDNA).

### [SEQ ID NO: 3]

This shows the base sequence of the primer used in EXAMPLE 1.

### [SEQ ID NO: 4]

This shows the base sequence of the primer used in EXAMPLE 1.

### [SEQ ID NO: 5]

This shows the amino acid sequence of 109 amino acid residues in the N-terminal of IRAP.

### [SEQ ID NO: 6]

This shows the base sequence of DNA encoding the amino acid sequence of 109 amino acid residues in the N-terminal of IRAP.

### [SEQ ID NO: 7]

This shows the amino acid sequence of amino acid residues from 468th to 510th of GLUT4.

### [SEQ ID NO: 8]

This shows the base sequence of DNA encoding the amino acid sequence of amino acid residues from 468th to 510th of GLUT4.

### [SEQ ID NO: 9]

This shows the amino acid sequence of rat VLCAD.

### [SEQ ID NO: 10]

This shows the amino acid sequence of mouse VLCAD.

### [SEQ ID NO: 11]

This shows the amino acid sequence of bovine VLCAD.

Transformant Escherichia coli DH5α/pTB2124, obtained in EXAMPLE 1 later described, harboring plasmid pTB2124 that contains the cDNA base sequence (SEQ ID NO: 2) of MD25 has been deposited in National Institute of Advanced Industrial Science and Technology, International Patent Organism Depositary (now-defunct Ministry of International Trade and Industry, Agency of Industrial Science and Technology, National Institute of Bioscience and Human Technology (NIBH)), located at Center No. 6, 1-1-1 Higasi, Tukuba, Ibaraki, 305-8566, Japan, under Accession Number FERM BP-7290 since September 4, 2000 and with the Institute for Fermentation, Osaka (IFO), located at 2-17-85, Zyuso-Honmati, Yodogawa-ku, Osaka shi, Osaka, 532-8686, Japan, under Accession Number IFO 16469 since August 24, 2000.

### EXAMPLES

The present invention is described in detail below with reference to EXAMPLES, but not intended to limit the scope of the present invention thereto. The gene manipulation procedures using Escherichia coli were performed according to the methods described in the Molecular Cloning.

### EXAMPLE 1: Cloning of cDNA encoding the IRAP-binding protein by yeast two-hybrid method.

cDNA encoding a protein bound to insulin responsive aminopeptidase (IRAP) was cloned by yeast two-hybrid method. As a rule, yeast two-hybrid method was conducted using Matchmaker two-hybrid system made by Clontech Laboratories, Inc.

DNA fragment encoding a polypeptide from 55th to 82nd amino acids residues of IRAP (Keller et al., J. Biol. Chem. 270: 23612-23618. 1995: SEQ ID NO: 5, amino acid numbers from 55 to 82) was chemically synthesized to insert into a plasmid pGBT9 (Clontech Laboratories, Inc.), which expresses a GAL4-DNA binding domain (GAL4-BD) under control of ADH1 promoter, in a fused form. This was named pBAIT-2, a bait vector. cDNA library used for screening was the cDNA library derived from a human skeletal muscle, by Clontech Laboratorires, Inc. The library has been constructed to express the library cDNA in a yeast cell in the fused form with GAL4 transcription-activating domain (GAL4-AD) under control of ADH1 promoter. As a host yeast, Saccharomyces cerevisiae Y190 was used. This yeast strain holds as the reporter gene, β-galastosidase (LacZ) and histidine synthesis gene (HIS3), that are controlled by TATA box and UAS (upstream activating sequences) of GAL1, on its chromosome.

pBAIT-2 (TRP1 marker) and the cDNA library plasmid (LEU2 marker) derived from the human skeletal muscle was introduced into S. cerevisiae yl90 and the transformant yeast, which has both plasmids and has expressed HIS3 being one of the reporter gene of the two-hybrid, was selected on an SD medium, which is a minimum essential medium, to which 60 mM 3-aminotriazole was added and tryptophane, leucine, and histidine were not added. The selected transformant colony was transferred to a nylon membrane by replica method, a cell wall of yeast was disrupted by freezing and thawing with liquid nitrogen, to carry out staining with X-Gal (5-bromo-4-chloro-β-D-galactoside), finally the strain presenting β-galactosidase activity was assigned to a primary candidate strain. By the method as described above, 10⁷ or more library cDNAs were screened to obtain candidate genes of 12 clones. A cell extract was prepared from these yeast cells by using Zymolyase (Seikagaku Kogyo Corp.), and Escherichia coli HB101, which is leucine-requiring strain, was transformed by using its DNA fraction.

Transformed Escherichia coli was plated on M9 medium not containing leucine and Escherichia coli strain having the library plasmid (LEU2 marker) was selected, and the plasmid was extracted from these strains. The extracted library plasmid and pMait-2, i. e., IRAP bait vector, was used to transform S. cerevisiae Y190 again, and thereafter histidine requirement and -β-galactosidase activity of the obtained transformant were tested resulting in yield of 5 clones showing reproducibility. From these clones, a clones showing strong β-galactosidase activity (MD25 strain) was selected. The base sequence of MD25 perfectly coincides with apart of human very-long chain acyl-CoA dehydrogenase (GenBank D43682: hereafter referred to as VLCAD). The coincided part was in a downstream from 36th amino acid of 655 amino acids (SEQ ID NO: 1) in a full length of VLCAD. The cDNA sequence containing the N-terminal of VLCAD was obtained by polymerase chain reaction (PCR) using the human skeletal muscle cDNA library as a template. The primer sequence used in PCR was presented below. 
(1) 5'-AGAGATTCGGAGATGCAGGCGGCT-3' (SEQ ID NO: 3)
(2) 5'-AGGGTAATGCCCACGCCAAGGTCA-3' (SEQ ID NO: 4)
 This PCR fragment and a partial fragment of VLCAD cloned by yeast two-hybrid method were ligated with each other in the site of restriction enzyme Sphl to make the full length MD25 cDNA (pTB2124).

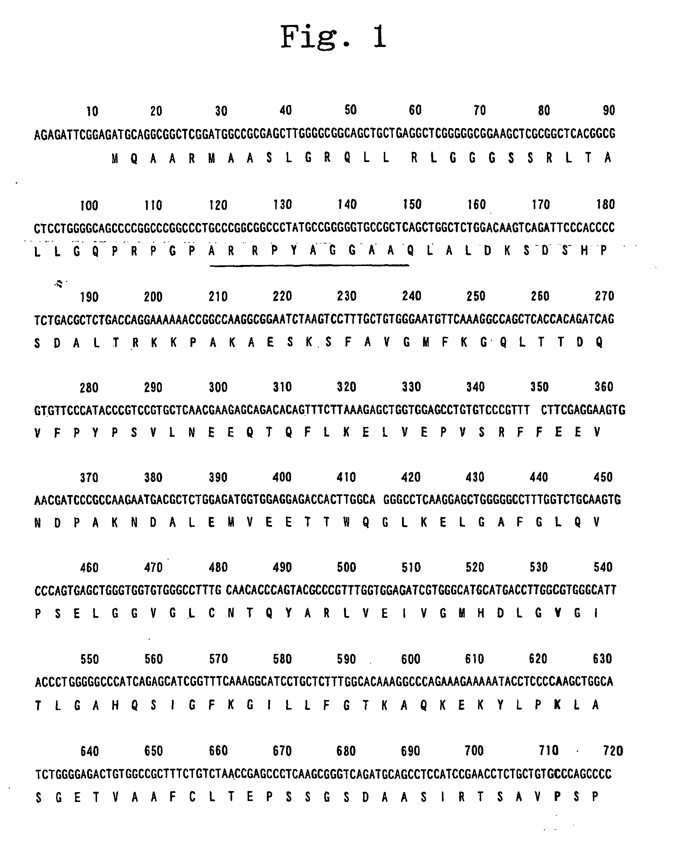

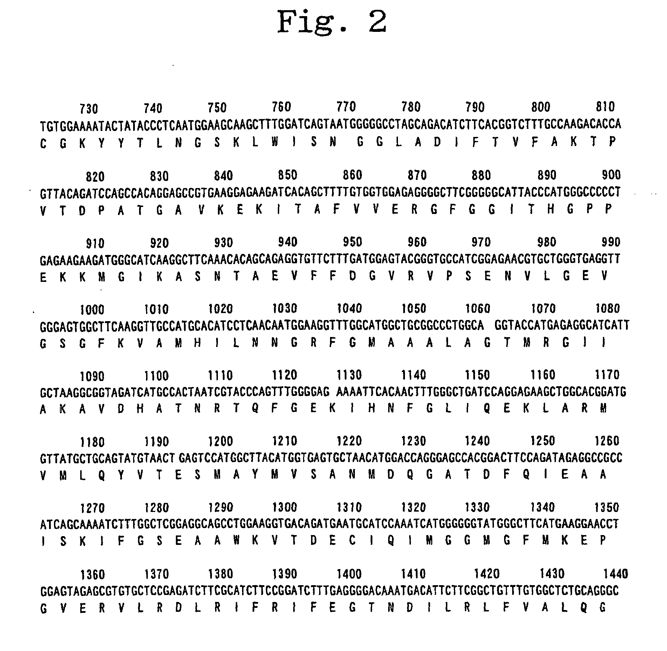

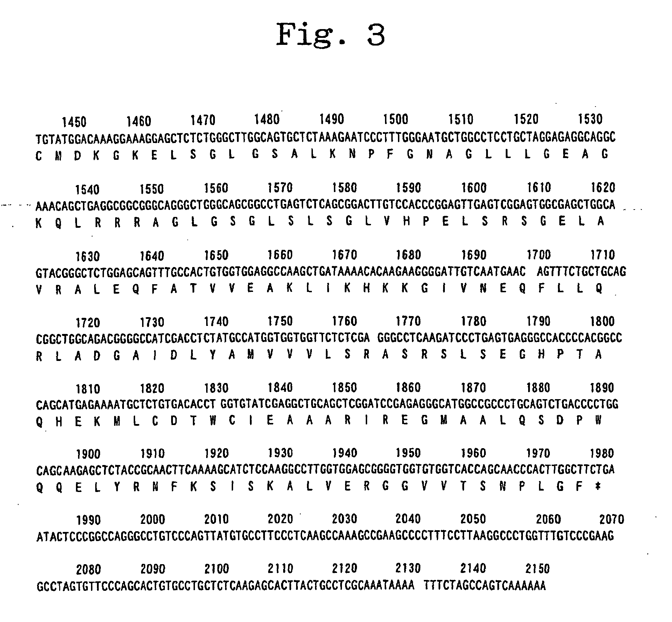

The base sequence of MD25 cDNA (SEQ ID NO: 2) and the amino acid sequence (SEQ ID NO: 1) were shown in Figs 1 to 3.

### EXAMPLE 2: Confirmation of a binding activity by measuring β-galastosidase activity.

To confirm the binding of MD25 to IRAP quantitatively, β-galactosidase activity was measured using CPRG (chlorophenol red-β-D-galactopyranoside) as a substrate.

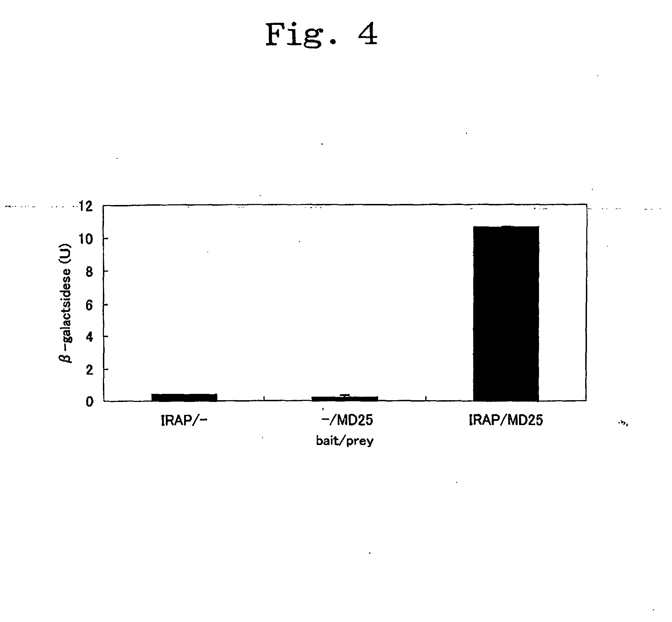

Yeast harboring both the vectors, Bait and prey, was cultured in liquid and cells were collected to disrupt cell walls by freezing and thawing using liquid nitrogen. CPRG was added to the disrupted cell suspension to measure absorbance of these samples at 578 nm as β-galactosidase activity. For a unit of β-galactosidase activity, 1 unit was defined as an enzyme activity of a single yeast cell to hydrolyze a 1 µmol of CPRG into chlorophenol red and galactoside for a minute. For a Bait sequence, IRAP (55-82; amino acid numbers 55th to 82nd of SEQ ID NO: 5) was used and for a prey sequence, the sequence (the sequence corresponding to that near 3' than the base number 118 of Figs 1 to 3) directly isolated from MD25 cDNA sequence by yeast two-hybrid method was used. In addition, as a negative control, a vector pGBT9 expressing GAL4-BD fused to no sequence to be used as bait, was used. S. cerevisiae Y190 was transformed by using plasmids having these sequences and β-galactosidase activity of the transformed yeast strain reconstructed was measured. The transformed strain having MD25 cDNA showed about 11 units of β-galactosidase activity if IRAP (55-82) is defined as bait. On the other hand, it showed almost no binding activity to the protein having GAL4-BD and lacking the bait sequence. A level of β-galactosidase activity was under a detection limit in an experiment using the strain lacking MD25 cDNA being the prey sequence (Fig. 4).

### EXAMPLE 3: Test of negative histidine requirement.

S. cerevisiae Y190, the parent strain, is the strain requiring histidine, however, in the case where bait couples to prey, HIS3 being one of reporter genes is expressed and thus, the yeast strain becomes negative in histidine requirement. The strain in which IRAP was transduced as bait and MD25 was as prey grew in SD culture medium (minimum essential medium,) to which 40 mM 3-aminotriazole was added and tryptophane, leucine, and histidine were not added. From this result, coupling of IRAP to MD25 was confirmed.

### EXAMPLE 4: Distribution of expression of MD25 mRNA in human tissues.

A expression distribution of MD25 mRNA in human tissues was detected by northern blotting. Specifically, northern blotting was conducted for human tissue poly(A)⁺ RNA (2 µg each) using MD25 cDNA (base numbers 479th to 2049th of SEQ ID NO: 3) as a probe. The nylon membrane, on which mRNA in each human tissue was transferred, was MTN blot (human 12 lane) available from Clontech Laboratories, Inc.

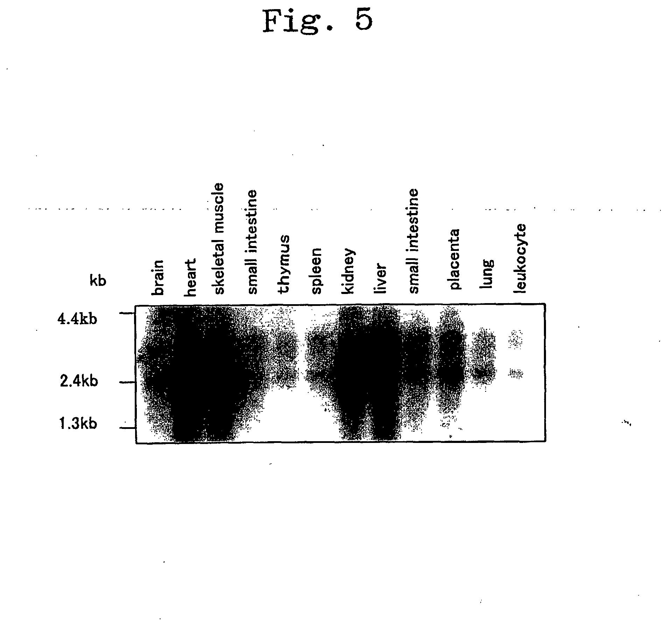

MD25 cDNA probe labeled with ³²P was hybridized and washed under a stringent condition and detected by an image analyzer BAS2000II (Fuji Film Corp.). As shown in Fig. 5, it was observed that MD25 mRNA expressed intensely in a heart, skeletal muscle, kidney and lever in an about 2.4 kb length.

### Industrial Applicability

The protein of the invention presents an intense expression in heart, skeletal muscle, kidney and lever.

Since the protein of the invention is bound to IRAP to raise a blood glucose level, it is useful as prophylactic and/or therapeutic agents for hypoglycemia.

The protein of the invention can be used for a screening method to inhibit binding of the protein of the invention to IRAP or GLUT4. A compound inhibiting binding of the protein of the invention to IRAP or GLUT4 is useful as prophylactic and/or therapeutic agents for diseases such as hyperglycemia and diabetes.

## Claims

1. A protein or a salt thereof having an amino acid sequence identical or substantially identical with the amino acid sequence represented by SEQ ID NO: 1, which is bound to an insulin responsive aminopeptidase or glucose transporter 4.

2. A pharmaceutical comprising the protein or the salt thereof according to claim 1.

3. The pharmaceutical comprising a DNA containing the DNA encoding the protein according to claim 1.

4. The pharmaceutical according to claim 2 or 3, being a binder to the insulin responsive aminopeptidase or glucose transporter 4.

5. The pharmaceutical according to claim 2 or 3, being a prophylactic and/or therapeutic agent for hypoglycemia.

6. A diagnostic agent comprising the DNA containing the DNA encoding the protein according to claim 1.

7. A method for prevention and treatment of hypoglycemia, which comprises administering an effective amount of the protein or the salt thereof according to claim 1 to a nonhuman mammal.

8. Use of the protein or the salt thereof according to claim 1 for manufacturing the prophylactic and/or therapeutic agent for hypoglycemia.

9. The pharmaceutical comprising an antisense DNA containing a base sequence or a segment thereof, which is complementary or substantially complementary to the base sequence of the DNA encoding the protein according to claim 1.

10. The pharmaceutical comprising an antibody against the protein or the salt thereof according to claim 1.

11. The pharmaceutical according to claim 9 or 10, being a prophylactic and/or therapeutic agent for hyperglycemia or diabetes.

12. A method for prevention and/or treatment of hyperglycemia or diabetes, which comprises administering the effective amount of the antibody against the protein or the salt thereof according to claim 1 to the nonhuman mammal.

13. Use of the antibody against the protein or the salt thereof according to claim 1 for manufacturing the prophylactic and/or therapeutic agent for hyperglycemia or diabetes.

14. A diagnostic agent comprising the antibody against the protein or the salt thereof according to claim 1.

15. A method for screening a compound having a hyperglycemic action or a hypoglycemic action, or the salt thereof, which comprises using the protein containing the amino acid sequence identical or substantially identical with the amino acid sequence represented by SEQ ID NO: 1,a partial peptide thereof, or the salt thereof.

16. The method for screening the compound having the hyperglycemic action or the hypoglycemic action, or the salt thereof, which comprises using the DNA containing the DNA, which encodes the protein containing the amino acid sequence identical or substantially identical with the amino acid sequence represented by SEQ ID NO: 1, or the partial peptide thereof.

17. A kit for screening the compound having the hyperglycemic action or the hypoglycemic action, or the salt thereof, wherein the kit comprises the protein containing the amino acid sequence identical or substantially identical with the amino acid sequencerepresented by SEQ ID NO: 1, the partial peptide thereof, or the salt thereof.

18. The kit for screening the compound having the hyperglycemic action or the hypoglycemic action, or the salt thereof, wherein the kit comprises the DNA containing the DNA, which encodes the protein containing the amino acid sequence identical or substantially identical with the amino acid sequence represented by SEQ ID NO: 1, or the partial peptide.

19. The compound having the hyperglycemic action, or the salt thereof obtainable by employing the screening method according to claim 15 or 16, or the screening kit according to claim 17 or 18.

20. A pharmaceutical comprising the compound according to claim 19 or the salt thereof.

21. The pharmaceutical according to claim 20, which is the prophylactic and/or therapeutic agent for hypoglycemia.

22. The compound having the hypoglycemic action, or the salt thereof obtainable by employing the screening method according to claim 15 or 16, or the screening kit according to claim 17 or 18.

23. A pharmaceutical comprising the compound according to claim 22, or the salt thereof

24. The pharmaceutical according to claim 23, which is a prophylactic and therapeutic agent for hyperglycemia or diabetes.

25. A method for screening the compound or the salt thereof enhancing or inhibiting binding the protein containing the amino acid sequence identical or substantially identical with the amino acid sequence represented by SEQ ID NO: 1, the partial peptide thereof, or the salt thereof to the insulin responsive aminopeptidase or glucose transporter 4, which comprises using the protein containing the amino acid sequence identical or substantially identical with the amino acid sequence represented by SEQ ID NO: 1,the partial peptide thereof, or the salt thereof.

26. The screening method according to claim 25, which comprises using a cell having an ability to produce the protein containing the amino acid sequence identical or substantially identical with the amino acid sequence represented by SEQ ID NO: 1,or the partial peptide thereof.

27. The screening method according to claim 25, which comprises adding a test compound to a complex between (1) the protein, containing the amino acid sequence identical or substantially identical with the amino acid sequence represented by SEQ ID NO: 1, the partial peptide thereof, or the salt thereof, and (2) the insulin responsive aminopeptidase or glucose transporter 4 labeled, of which one has been fixed on a solid phase, and detecting a free substance.

28. The screening method according to claim 25, which comprises culturing a cell transformed with (1) DNA encoding the partial peptide corresponding to a cytoplasmic domain of the insulin responsive aminopeptidase, or the partial peptide corresponding to a intracellular domain of glucose transporter 4, with which a reporter gene binding domain has been fused, and (2) DNA encoding the protein or the partial peptide thereof containing the amino acid sequence identical or substantially identical with the amino acid sequence represented by SEQ ID NO: 1, with which a reporter gene transcription-active domain has been fused, in a presence and absence of the test compound and detecting expression of a reporter gene in both the cases.

29. The screening method according to claim 28, wherein the partial peptide corresponding to the cytoplasmic domain of the insulin responsive aminopeptidase is the partial peptide containing the amino acid sequence from 55th to 82nd position of the amino acid sequence represented by SEQ ID NO: 5 and the partial peptide corresponding to the cytoplasmic domain of glucose transporter 4 is the partial peptide containing the amino acid sequence represented by SEQ ID NO: 7.

30. A kit for screening the compound or the salt thereof, which contains the protein, the partial peptide thereof, or the salt thereof containing the amino acid sequence identical or substantially identical with the amino acid sequence represented by SEQ ID NO: 1, inhibiting the binding of the protein, the partial peptide thereof, or the salt thereof containing the amino acid sequence identical or substantially identical with the amino acid sequence represented by SEQ ID NO: 1, to the insulin responsive aminopeptidase or glucose transporter 4.

31. A compound or the salt thereof, which is obtainable by using the screening method according to claim 25 to 29 or the screening kit according to claim 30, inhibiting the binding of the protein, the partial peptide thereof, or the salt thereof containing the amino acid sequence identical or substantially identical with the amino acid sequence represented by SEQ ID NO: 1, to the insulin responsive aminopeptidase or glucose transporter 4.

32. A pharmaceutical comprising the compound or the salt thereof according to claim 31.

33. The pharmaceutical according to claim 32, which is a prophylactic and/or therapeutic agent for hyperglycemia or diabetes.

34. A method for prevention and treatment of hyperglycemia or diabetes, which comprises administering the effective amount of the compound or the salt thereof according to claim 31 to the nonhuman mammal.

35. Use of the compound or the salt thereof according to claim 31, for manufacturing the prophylactic and/or therapeutic agent for hyperglycemia or diabetes.

36. A compound or the salt thereof, which is obtainable by using the screening method according to claim 25 to 29, or the screening kit according to claim 30, enhancing the binding of the protein, the partial peptide thereof, or the salt thereof containing the amino acid sequence identical or substantially identical with the amino acid sequence represented by SEQ ID NO: 1, to the insulin responsive aminopeptidase or glucose transporter 4.

37. A pharmaceutical comprising the compound or the salt thereof according to claim 36.

38. The pharmaceutical according to claim 37, which is a prophylactic and/or therapeutic agent for hypoglycemia.

39. A method for prevention and treatment of hypoglycemia, which comprises administering the effective amount of the compound or the salt thereof according to claim 36 to the nonhuman mammal.

40. Use of the compound or the salt thereof according to claim 31, for manufacturing the prophylactic and/or therapeutic agent for hypoglycemia.

41. A method for screening the compound enhancing or suppressing the expression of the protein, or the salt thereof, which comprises using the protein or the salt thereof containing the amino acid sequence identical or substantially identical with the amino acid sequence represented by SEQ ID NO: 1.

42. The screening method according to claim 41, which comprises culturing the cell transformed with DNA, which is prepared by fusing the reporter gene with a transcription regulating region of DNA encoding the protein containing the amino acid sequence identical or substantially identical with the amino acid sequence represented by SEQ ID NO: 1 in the presence and absence of the test compound and detecting expression of the reporter gene in each case.

43. A kit for screening the compound enhancing or suppressing the expression of the protein, or the salt thereof, wherein the kit comprises the protein or the salt thereof containing the amino acid sequence identical or substantially identical with the amino acid sequence represented by SEQ ID NO: 1.

44. A compound or the salt thereof, which is obtainable by using the screening method according to claim 41 or 42, or the screening kit according to claim 43, suppressing expression of the protein containing the amino acid sequence identical or substantially identical with the amino acid sequence represented by SEQ ID NO: 1.

45. A pharmaceutical comprising the compound or the salt thereof according to claim 44.

46. The pharmaceutical according to claim 45, which is the prophylactic and/or therapeutic agent for hyperglycemia or diabetes.

47. A compound or the salt thereof, which is obtainable by using the screening method according to claim 41 or 42, or the screening kit according to claim 43, enhancing expression of the protein containing the amino acid sequence identical or substantially identical with the amino acid sequence represented by SEQ ID NO: 1.

48. The pharmaceutical comprising the compound or the salt thereof according to claim 47.

49. The pharmaceutical according to claim 48, which is a prophylactic and/or therapeutic agent for hypoglycemia.

50. A method for prevention and treatment of hypoglycemia, which comprises administering the effective amount of the compound or the salt thereof according to claim 47 to the nonhuman mammal.

51. Use of the compound or the salt thereof according to claim 47 for manufacturing the prophylactic and/or therapeutic agent for hypoglycemia.


SEQUENCE LISTING

<110> Takeda Chemical Industries, Ltd.

<120> IRAP Binding Protein

<130> P2001-143PCT

<150> JP 2000-254263
<151> 2000-08-21

<150> JP 2000-276633
<151> 2000-09-07

<160> 11

<210> 1
<211> 655
<212> PRT
<213> Human

<400> 1

<210> 2
<211> 1965
<212> DNA
<213> Human

<400> 2
   atgcaggcgg ctcggatggc cgcgagcttg gggcggcagc tgctgaggct cgggggcgga 60
   agctcgcggc tcacggcgct cctggggcag ccccggcccg gccctgcccg gcggccctat 120
   gccgggggtg ccgctcagct ggctctggac aagtcagatt cccacccctc tgacgctctg 180
   accaggaaaa aaccggccaa ggcggaatct aagtcctttg ctgtgggaat gttcaaaggc 240
   cagctcacca cagatcaggt gttcccatac ccgtccgtgc tcaacgaaga gcagacacag 300
   tttcttaaag agctggtgga gcctgtgtcc cgtttcttcg aggaagtgaa cgatcccgcc 360
   aagaatgacg ctctggagat ggtggaggag accacttggc agggcctcaa ggagctgggg 420
   gcctttggtc tgcaagtgcc cagtgagctg ggtggtgtgg gcctttgcaa cacccagtac 480
   gcccgtttgg tggagatcgt gggcatgcat gaccttggcg tgggcattac cctgggggcc 540
   catcagagca tcggtttcaa aggcatcctg ctctttggca caaaggccca gaaagaaaaa 600
   tacctcccca agctggcatc tggggagact gtggccgctt tctgtctaac cgagccctca 660
   agcgggtcag atgcagcctc catccgaacc tctgctgtgc ccagcccctg tggaaaatac 720
   tataccctca atggaagcaa gctttggatc agtaatgggg gcctagcaga catcttcacg 780
   gtctttgcca agacaccagt tacagatcca gccacaggag ccgtgaagga gaagatcaca 840
   gcttttgtgg tggagagggg cttcgggggc attacccatg ggccccctga gaagaagatg 900
   ggcatcaagg cttcaaacac agcagaggtg ttctttgatg gagtacgggt gccatcggag 960
   aacgtgctgg gtgaggttgg gagtggcttc aaggttgcca tgcacatcct caacaatgga 1020
   aggtttggca tggctgcggc cctggcaggt accatgagag gcatcattgc taaggcggta 1080
   gatcatgcca ctaatcgtac ccagtttggg gagaaaattc acaactttgg gctgatccag 1140
   gagaagctgg cacggatggt tatgctgcag tatgtaactg agtccatggc ttacatggtg 1200
   agtgctaaca tggaccaggg agccacggac ttccagatag aggccgccat cagcaaaatc 1260
   tttggctcgg aggcagcctg gaaggtgaca gatgaatgca tccaaatcat ggggggtatg 1320
   ggcttcatga aggaacctgg agtagagcgt gtgctccgag atcttcgcat cttccggatc 1380
   tttgagggga caaatgacat tcttcggctg tttgtggctc tgcagggctg tatggacaaa 1440
   ggaaaggagc tctctgggct tggcagtgct ctaaagaatc cctttgggaa tgctggcctc 1500
   ctgctaggag aggcaggcaa acagctgagg cggcgggcag ggctgggcag cggcctgagt 1560
   ctcagcggac ttgtccaccc ggagttgagt cggagtggcg agctggcagt acgggctctg 1620
   gagcagtttg ccactgtggt ggaggccaag ctgataaaac acaagaaggg gattgtcaat 1680
   gaacagtttc tgctgcagcg gctggcagac ggggccatcg acctctatgc catggtggtg 1740
   gttctctcga gggcctcaag atccctgagt gagggccacc ccacggccca gcatgagaaa 1800
   atgctctgtg acacctggtg tatcgaggct gcagctcgga tccgagaggg catggccgcc 1860
   ctgcagtctg acccctggca gcaagagctc taccgcaact tcaaaagcat ctccaaggcc 1920
   ttggtggagc ggggtggtgt ggtcaccagc aacccacttg gcttc 1965

<210> 3
<211> 24
<212> DNA
<213> Artificial Sequence

<220>
<223> Primer

<400> 3
agagattcgg agatgcaggc ggct        24

<210> 4
<211> 24
<212> DNA
<213> Artificial Sequence

<220>
<223> Primer

<400> 4
agggtaatgc ccacgccaag gtca        24

<210> 5
<211> 109
<212> PRT
<213> Human

<400> 5
Met Glu Thr Phe Thr Asn Asp Arg Leu Gln Leu Pro Arg Asn Met Ile

1                   5                   10                  15
Glu Asn Ser Met Phe Glu Glu Glu Pro Asp Val Val Asp Leu Ala Lys
                20                  25                  30
Glu Pro Cys Leu His Pro Leu Glu Pro Asp Glu Val Glu Tyr Glu Pro
            35                  40                  45
Arg Gly Ser Arg Leu Leu Val Arg Gly Leu Gly Glu His Glu Met Asp
        50                  55                  60
Glu Asp Glu Glu Asp Tyr Glu Ser Ser Ala Lys Leu Leu Gly Met Ser
65                  70                  75                  80
Phe Met Asn Arg Ser Ser Gly Leu Arg Asn Ser Ala Thr Gly Tyr Arg
                    85                  90                  95
Gln Ser Pro Asp Gly Thr Cys Ser Val Pro Ser Ala Arg
                100                 105

<210> 6
<211> 327
<212> DNA
<213> Human

<400> 6
atggagacct ttaccaatga tcgacttcag cttccaagga atatgattga aaacagcatg    60
tttgaagaag aaccagatgt ggtagattta gccaaagaac cttgtttaca tcctctggaa   120
cctgatgaag ttgaatatga gccccgaggt tcgaggcttc tggtgagagg tcttggtgag   180
catgagatgg atgaggatga agaggattat gagtcatctg ccaagctgct gggcatgtcc   240
ttcatgaaca gaagctcagg ccttcggaac agtgcaacag gctacaggca gagtccagat   300
gggacttgtt cagtaccctc tgccagg                                       327

<210> 7
<211> 43

<212> PRT
<213> Human

<400> 7
Lys Val Pro Glu Thr Arg Gly Arg Thr Phe Asp Gln Ile Ser Ala Ala
1               5                   10                  15
Phe Arg Arg Thr Pro Ser Leu Leu Glu Gln Glu Val Lys Pro Ser Thr
            20                  25                  30
Glu Leu Glu Tyr Leu Gly Pro Asp Glu Asn Asp
        35                  40

<210> 8
<211> 129
<212> DNA
<213> Human

<400> 8
aaagtgcctg aaaccagagg ccggacgttt gaccagatct cagctgcctt ccgacggaca    60
ccttcccttt tagagcagga ggtgaaaccc agtacagaac ttgaatactt agggccagat   120
gagaatgac                                                            129

<210> 9
<211> 653
<212> PRT
<213> Rat

<400> 9
Met Gln Ser Ala Arg Met Thr Pro Ser Val Gly Arg Gln Leu Leu Arg
1               5                   10                  15

Leu Gly Ala Arg Ser Ser Arg Ser Ala Ala Leu Gln Gly Gln Pro Arg
            20                  25                  30
Pro Thr Ser Ala Gln Arg Leu Tyr Ala Ser Glu Ala Thr Gln Ala Val
        35                  40                  45
Leu Glu Lys Pro Glu Thr Leu Ser Ser Asp Ala Ser Thr Arg Glu Lys
    50                  55                  60
Pro Ala Arg Ala Glu Ser Lys Ser Phe Ala Val Gly Met Phe Lys Gly
65                  70                  75                  80
Gln Leu Thr Thr Asp Gln Val Phe Pro Tyr Pro Ser Val Leu Asn Glu
                85                  90                  95
Gly Gln Thr Gln Phe Leu Lys Glu Leu Val Gly Pro Val Ala Arg Phe
            100                 105                 110
Phe Glu Glu Val Asn Asp Pro Ala Lys Asn Asp Ser Leu Glu Lys Val
        115                 120                 125
Glu Glu Asp Thr Leu Gln Gly Leu Lys Glu Leu Gly Ala Phe Gly Leu
    130                 135                 140
Gln Val Pro Ser Glu Leu Gly Gly Leu Gly Leu Ser Asn Thr Gln Tyr
145                 150                 155                 160
Ala Arg Leu Ala Glu Ile Val Gly Met His Asp Leu Gly Val Ser Val
                165                 170                 175
Thr Leu Gly Ala His Gln Ser Ile Gly Phe Lys Gly Ile Leu Leu Tyr
            180                 185                 190
Gly Thr Lys Ala Gln Lys Glu Lys Tyr Leu Pro Arg Val Ala Ser Gly
        195                 200                 205
Gln Ala Leu Ala Ala Phe Cys Leu Thr Glu Pro Ser Ser Gly Ser Asp
    210                 215                 220
Val Ala Ser Ile Arg Ser Ser Ala Val Pro Ser Pro Cys Gly Lys Tyr
225                 230                 235                 240
Tyr Thr Leu Asn Gly Ser Lys Ile Trp Ile Ser Asn Gly Gly Leu Ala

                245                 250                 255
Asp Ile Phe Thr Val Phe Ala Lys Thr Pro Ile Lys Asp Ala Ala Thr
            260                 265                 270
Gly Ala Val Lys Glu Lys Ile Thr Ala Phe Val Val Glu Arg Ser Phe
        275                 280                 285
Gly Gly Val Thr His Gly Leu Pro Glu Lys Lys Met Gly Ile Lys Ala
    290                 295                 300
Ser Asn Thr Ser Glu Val Tyr Phe Asp Gly Val Lys Val Pro Ala Glu
305                 310                 315                 320
Asn Val Leu Gly Glu Val Gly Asp Gly Phe Lys Val Ala Val Asn Ile
                325                 330                 335
Leu Asn Asn Gly Arg Phe Gly Met Ala Ala Thr Leu Ala Gly Thr Met
            340                 345                 350
Lys Ala Ile Ile Ala Lys Ala Val Asp His Ala Thr Asn Arg Thr Gln
        355                 360                 365
Phe Gly Asp Lys Ile His Asn Phe Gly Val Ile Gln Glu Lys Leu Ala
    370                 375                 380
Arg Met Ala Ile Leu Gln Tyr Val Thr Glu Ser Met Ala Tyr Met Leu
385                 390                 395                 400
Ser Ala Asn Met Asp Gln Gly Phe Lys Asp Phe Gln Ile Glu Ala Ala
                405                 410                 415
Ile Ser Lys Ile Phe Gly Ser Glu Ala Ala Trp Lys Val Thr Asp Glu
            420                 425                 430
Cys Ile Gln Ile Met Gly Gly Met Gly Phe Met Lys Glu Pro Gly Val
        435                 440                 445
Glu Arg Val Leu Arg Asp Ile Arg Ile Phe Arg Ile Phe Glu Gly Thr
    450                 455                 460
Asn Asp Ile Leu Arg Leu Phe Val Ala Leu Gln Gly Cys Met Asp Lys
465                 470                 475                 480

Gly Lys Glu Leu Thr Gly Leu Gly Asn Ala Leu Lys Asn Pro Leu Gly
                485                 490                 495
Asn Val Gly Leu Leu Ile Gly Glu Ala Ser Lys Gln Leu Arg Arg Arg
            500                 505                 510
Thr Gly Ile Gly Ser Gly Leu Ser Leu Ser Gly Ile Val His Pro Glu
        515                 520                 525
Leu Ser Arg Ser Gly Glu Leu Ala Val Gln Ala Leu Glu Gln Phe Ala
    530                 535                 540
Thr Val Val Glu Ala Lys Leu Met Lys His Lys Lys Gly Ile Val Asn
545                 550                 555                 560
Glu Gln Phe Leu Leu Gln Arg Leu Ala Asp Gly Ala Ile Asp Leu Tyr
                565                 570                 575
Ala Met Val Val Val Leu Ser Arg Ala Ser Arg Ser Leu Ser Glu Gly
            580                 585                 590
Tyr Pro Thr Ala Gln His Glu Lys Met Leu Cys Asp Ser Trp Cys Ile
        595                 600                 605
Glu Ala Ala Thr Arg Ile Arg Glu Asn Met Ala Ser Leu Gln Ser Asn
    610                 615                 620
Pro Gln Gln Gln Glu Leu Phe Arg Asn Phe Arg Ser Ile Ser Lys Ala
625                 630                 635                 640
Met Val Glu Asn Gly Gly Leu Val Thr Ser Asn Pro Leu
                645                 650             653

<210> 10
<211> 655
<212> PRT
<213> Mouse

<400> 10

Met Gln Ser Ala Arg Met Thr Pro Ser Val Gly Arg Gln Leu Leu Arg
1               5                   10                  15
Leu Gly Ala Arg Ser Ser Arg Ser Thr Thr Val Leu Gln Gly Gln Pro
            20                  25                  30
Arg Pro Ile Ser Ala Gln Arg Leu Tyr Ala Arg Glu Ala Thr Gln Ala
        35                  40                  45
Val Leu Asp Lys Pro Glu Thr Leu Ser Ser Asp Ala Ser Thr Arg Glu
    50                  55                  60
Lys Pro Ala Arg Ala Glu Ser Lys Ser Phe Ala Val Gly Met Phe Lys
65                  70                  75                  80
Gly Gln Leu Thr Ile Asp Gln Val Phe Pro Tyr Pro Ser Val Leu Ser
                85                  90                  95
Glu Glu Gln Ala Gln Phe Leu Lys Glu Leu Val Gly Pro Val Ala Arg
            100                 105                 110
Phe Phe Glu Glu Val Asn Asp Pro Ala Lys Asn Asp Ala Leu Glu Lys
        115                 120                 125
Val Glu Asp Asp Thr Leu Gln Gly Leu Lys Glu Leu Gly Ala Phe Gly
    130                 135                 140
Leu Gln Val Pro Ser Glu Leu Gly Gly Leu Gly Leu Ser Asn Thr Gln
145                 150                 155                 160
Tyr Ala Arg Leu Ala Glu Ile Val Gly Met His Asp Leu Gly Val Ser
                165                 170                 175
Val Thr Leu Gly Ala His Gln Ser Ile Gly Phe Lys Gly Ile Leu Leu
            180                 185                 190
Tyr Gly Thr Lys Ala Gln Arg Glu Lys Tyr Leu Pro Arg Val Ala Ser
        195                 200                 205
Gly Gln Ala Leu Ala Ala Phe Cys Leu Thr Glu Pro Ser Ser Gly Ser
    210                 215                 220
Asp Val Ala Ser Ile Arg Ser Ser Ala Ile Pro Ser Pro Cys Gly Lys

225                 230                 235                 240
Tyr Tyr Thr Leu Asn Gly Ser Lys Ile Trp Ile Ser Asn Gly Gly Leu
                245                 250                 255
Ala Asp Ile Phe Thr Val Phe Ala Lys Thr Pro Ile Lys Asp Ala Ala
            260                 265                 270
Thr Gly Ala Val Lys Glu Lys Ile Thr Ala Phe Val Val Glu Arg Ser
        275                 280                 285
Phe Gly Gly Val Thr His Gly Leu Pro Glu Lys Lys Met Gly Ile Lys
    290                 295                 300
Ala Ser Asn Thr Ser Glu Val Tyr Phe Asp Gly Val Lys Val Pro Ser
305                 310                 315                 320
Glu Asn Val Leu Gly Glu Val Gly Asp Gly Phe Lys Val Ala Val Asn
                325                 330                 335
Ile Leu Asn Asn Gly Arg Phe Gly Met Ala Ala Thr Leu Ala Gly Thr
            340                 345                 350
Met Lys Ser Leu Ile Ala Lys Ala Val Asp His Ala Thr Asn Arg Thr
        355                 360                 365
Gln Phe Gly Asp Lys Ile His Asn Phe Gly Val Ile Gln Glu Lys Leu
    370                 375                 380
Ala Arg Met Ala Ile Leu Gln Tyr Val Thr Glu Ser Met Ala Tyr Met
385                 390                 395                 400
Leu Ser Ala Asn Met Asp Gln Gly Phe Lys Asp Phe Gln Ile Glu Ala
                405                 410                 415
Ala Ile Ser Lys Ile Phe Cys Ser Glu Ala Ala Trp Lys Val Ala Asp
            420                 425                 430
Glu Cys Ile Gln Ile Met Gly Gly Met Gly Phe Met Lys Glu Pro Gly
        435                 440                 445
Val Glu Arg Val Leu Arg Asp Ile Arg Ile Phe Arg Ile Phe Glu Gly
    450                 455                 460

Ala Asn Asp Ile Leu Arg Leu Phe Val Ala Leu Gln Gly Cys Met Asp
465                 470                 475                 480
Lys Gly Lys Glu Leu Thr Gly Leu Gly Asn Ala Leu Lys Asn Pro Phe
                485                 490                 495
Gly Asn Val Gly Leu Leu Met Gly Glu Ala Gly Lys Gln Leu Arg Arg
            500                 505                 510
Arg Thr Gly Ile Gly Ser Gly Leu Ser Leu Ser Gly Ile Val His Pro
        515                 520                 525
Glu Leu Ser Arg Ser Gly Glu Leu Ala Val Gln Ala Leu Asp Gln Phe
    530                 535                 540
Ala Thr Val Val Glu Ala Lys Leu Val Lys His Lys Lys Gly Ile Val
545                 550                 555                 560
Asn Glu Gln Phe Leu Leu Gln Arg Leu Ala Asp Gly Ala Ile Asp Leu
                565                 570                 575
Tyr Ala Met Val Val Val Leu Ser Arg Ala Ser Arg Ser Leu Ser Glu
            580                 585                 590
Gly Tyr Pro Thr Ala Gln His Glu Lys Met Leu Cys Asp Ser Trp Cys
        595                 600                 605
Ile Glu Ala Ala Thr Arg Ile Arg Glu Asn Met Ala Ser Leu Gln Ser
    610                 615                 620
Ser Pro Gln His Gln Glu Leu Phe Arg Asn Phe Arg Ser Ile Ser Lys
625                 630                 635                 640
Ala Met Val Glu Asn Gly Gly Leu Val Thr Gly Asn Pro Leu Gly
                645                 650                 655

<210> 11
<211> 655
<212> PRT
<213> Bovine

<400> 11
Met Gln Ala Ala Arg Met Thr Ala Ser Leu Gly Arg Thr Leu Leu Arg
1               5                   10                  15
Leu Arg Gly Val Ser Ser Trp Pro Gly Glu Leu Leu Gly Gln Pro Arg
            20                  25                  30
Pro Gly Pro Ala Pro Arg Pro Tyr Ala Ser Gly Val Ala Gln Ala Ala
        35                  40                  45
Val Asp Gln Ser Asp Ser Gln Pro Ser Glu Ala Ser Thr Arg Glu Lys
    50                  55                  60
Arg Ala Asn Ser Val Ser Lys Ser Phe Ala Val Gly Thr Phe Lys Gly
65                  70                  75                  80
Gln Leu Thr Thr Asp Gln Val Phe Pro Tyr Pro Ser Val Leu Asn Glu
                85                  90                  95
Asp Gln Thr Gln Phe Leu Lys Glu Leu Val Gly Pro Val Thr Arg Phe
            100                 105                 110
Phe Glu Glu Val Asn Asp Ala Ala Lys Asn Asp Met Leu Glu Arg Val
        115                 120                 125
Glu Glu Thr Thr Met Gln Gly Leu Lys Glu Leu Gly Ala Phe Gly Leu
    130                 135                 140
Gln Val Pro Asn Glu Leu Gly Gly Val Gly Leu Cys Asn Thr Gln Tyr
145                 150                 155                 160
Ala Arg Leu Val Glu Ile Val Gly Met Tyr Asp Leu Gly Val Gly Ile
                165                 170                 175
Val Leu Gly Ala His Gln Ser Ile Gly Phe Lys Gly Ile Leu Leu Phe
            180                 185                 190
Gly Thr Lys Ala Gln Lys Glu Lys Tyr Leu Pro Lys Leu Ala Ser Gly
        195                 200                 205
Glu Thr Ile Ala Ala Phe Cys Leu Thr Glu Pro Ser Ser Gly Ser Asp

    210                 215                 220
Ala Ala Ser Ile Arg Ser Ser Ala Val Pro Ser Pro Cys Gly Lys Tyr
225                 230                 235                 240
Tyr Thr Leu Asn Gly Ser Lys Ile Trp Ile Ser Asn Gly Gly Leu Ala
                245                 250                 255
Asp Ile Phe Thr Val Phe Ala Lys Thr Pro Val Thr Asp Thr Ala Thr
            260                 265                 270
Gly Ala Val Lys Glu Lys Ile Thr Ala Phe Val Val Glu Arg Ser Phe
        275                 280                 285
Gly Gly Val Thr His Gly Pro Pro Glu Lys Lys Met Gly Ile Lys Ala
    290                 295                 300
Ser Asn Thr Ala Glu Val Tyr Phe Asp Gly Val Arg Val Pro Ala Glu
305                 310                 315                 320
Asn Val Leu Gly Glu Val Gly Gly Gly Phe Lys Val Ala Met His Ile
                325                 330                 335
Leu Asn Asn Gly Arg Phe Gly Met Ala Ala Ala Leu Ala Gly Thr Met
            340                 345                 350
Lys Gly Ile Ile Ala Lys Ala Val Asp His Ala Ala Asn Arg Thr Gln
        355                 360                 365
Phe Gly Glu Lys Ile His Asn Phe Gly Leu Ile Gln Glu Lys Leu Ala
    370                 375                 380
Arg Met Ala Met Leu Gln Tyr Val Thr Glu Ser Met Ala Tyr Met Val
385                 390                 395                 400
Ser Ala Asn Met Asp Gln Gly Ser Thr Asp Phe Gln Ile Glu Ala Ala
                405                 410                 415
Ile Ser Lys Ile Phe Gly Ser Glu Ala Ala Trp Lys Val Thr Asp Glu
            420                 425                 430
Cys Ile Gln Ile Met Gly Gly Met Gly Phe Met Lys Glu Pro Gly Val
        435                 440                 445

Glu Arg Val Leu Arg Asp Leu Arg Ile Phe Arg Ile Phe Glu Gly Thr
    450                 455                 460
Asn Asp Ile Leu Arg Leu Phe Val Ala Leu Gln Gly Cys Met Asp Lys
465                 470                 475                 480
Gly Lys Glu Leu Ser Gly Leu Gly Asn Ala Leu Lys Asn Pro Phe Gly
                485                 490                 495
Asn Ala Gly Leu Leu Leu Gly Glu Ala Gly Lys Gln Leu Arg Arg Arg
            500                 505                 510
Ala Gly Leu Gly Ser Gly Leu Ser Leu Ser Gly Ile Val His Gln Glu
        515                 520                 525
Leu Ser Arg Ser Gly Glu Leu Ala Val Gln Ala Leu Glu Gln Phe Ala
    530                 535                 540
Thr Val Val Glu Ala Lys Leu Ile Lys His Lys Lys Asp Ile Ile Asn
545                 550                 555                 560
Glu Gln Phe Leu Leu Gln Arg Leu Ala Asp Ser Ala Ile Asp Leu Tyr
                565                 570                 575
Ala Met Val Val Val Leu Ser Arg Ala Ser Arg Ser Leu Ser Glu Gly
            580                 585                 590
His Pro Thr Ala Gln His Glu Lys Met Leu Cys Asp Ser Trp Cys Ile
        595                 600                 605
Glu Ala Ala Ala Arg Ile Arg Glu Asn Met Thr Ala Leu Gln Ser Asp
    610                 615                 620
Pro Gln Gln Gln Glu Leu Phe Arg Asn Phe Lys Ser Ile Ser Lys Ala
625                 630                 635                 640
Leu Val Glu Arg Gly Gly Val Val Thr Ser Asn Pro Leu Gly Phe
                645                 650                 655